(19) Europäisches Patentamt / European Patent Office / Office européen des brevets

(11) **EP 4 467 161 A1**

(12) **EUROPEAN PATENT APPLICATION**
published in accordance with Art. 153(4) EPC

(43) Date of publication:
**27.11.2024 Bulletin 2024/48**

(21) Application number: **22921507.4**

(22) Date of filing: **16.09.2022**

(51) International Patent Classification (IPC):
**A61K 49/18** (2006.01)   **A61K 49/14** (2006.01)
**A61K 49/12** (2006.01)   **B82Y 5/00** (2011.01)
**B82Y 40/00** (2011.01)

(52) Cooperative Patent Classification (CPC):
**A61K 49/12; A61K 49/14; A61K 49/18; B82Y 5/00; B82Y 40/00**

(86) International application number:
**PCT/CN2022/119469**

(87) International publication number:
**WO 2023/138080 (27.07.2023 Gazette 2023/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.01.2022 CN 202210072699**

(71) Applicants:
• **SUZHOU ZHECI PHARMACEUTICAL TECHNOLOGY CO., LTD.**
  **Suzhou, Jiangsu 215011 (CN)**

• **South Medical University**
  **Baiyun District**
  **Guangzhou, Guangdong 510515 (CN)**

(72) Inventors:
• **SHEN, Zheyu**
  **Suzhou, Jiangsu 215011 (CN)**
• **LU, Xuanyi**
  **Suzhou, Jiangsu 215011 (CN)**

(74) Representative: **Hafner & Kohl PartmbB**
**Schleiermacherstraße 25**
**90491 Nürnberg (DE)**

(54) **MAGNETIC FERROFERRIC OXIDE NANOPARTICLE, AND PREPARATION METHOD THEREFOR AND USE THEREOF**

(57)    The present invention discloses a magnetic ferroferric oxide nanoparticle, and a preparation method therefor and the use thereof. The magnetic ferroferric oxide nanoparticle contains ferroferric oxide and a hydrophilic macromolecule, wherein the ferroferric oxide and the hydrophilic macromolecule are in at least one of the following relationships (1) and (2): (1) the hydrophilic macromolecule is adsorbed on the surface of the ferroferric oxide; and (2) the ferroferric oxide and the hydrophilic macromolecule are in the state of mutual embedding or occlusion. In the present invention, the hydrophilic macromolecule is used as a stabilizer, and ferrous ions and ferric ions form the magnetic ferroferric oxide nanoparticle by means of coprecipitation; and the magnetic ferroferric oxide nanoparticle has a relatively high longitudinal magnetic relaxation rate $r_1$, a relatively low transverse/longitudinal magnetic relaxation rate ratio ($r_2/r_1$), good water solubility, high stability, and good biocompatibility, and can be used as a contrast agent for T1-weighted magnetic resonance imaging (MRI) to improve the contrast and sensitivity of MRI.

**Description**

**TECHNICAL FIELD**

The present disclosure relates to the technical field of biomedical materials, and particularly to a magnetic ferroferric oxide nanoparticle, and a preparation method therefor and use thereof.

**BACKGROUND**

Magnetic resonance imaging (MRI) has a good imaging effect on soft tissues and may effectively distinguish lesions from healthy tissues, thus being widely used in clinical diagnosis. An MRI contrast agent can improve the contrast and sensitivity of MRI. At present, the contrast agent is used in more than 40% of MRI clinical diagnosis, and the MRI contrast agent has become an important part of magnetic resonance imaging. The MRI contrast agent may react with hydrogen proton, thus shortening longitudinal relaxation time ($T_1$) or transverse relaxation time ($T_2$) of the proton. The MRI contrast agent may be divided into two types according to imaging effects: $T_1$-weighted MRI contrast agent (positive contrast agent), which may generate a bright signal; $T_2$-weighted MRI contrast agent (negative contrast agent), which may generate a dark signal. Compared with the $T_2$ contrast agent, the $T_1$ contrast agent may generate the bright signal, which is more conducive to lesion diagnosis by clinicians, so that the $T_1$ contrast agent is mostly used in clinic.

An $r_1$ value and an $r_2/r_1$ ratio are two important parameters to characterize the imaging effect of the MRI contrast agent. The higher the $r_1$ value of the $T_1$ contrast agent is, the better the effect is, and the lower the $r_2/r_1$ ratio is, the better the effect is; and similarly, the higher the $r_2$ value of the $T_2$ contrast agent is, the better the effect is, and the higher the $r_2/r_1$ ratio is, the better the effect is. Generally, the $T_1$ contrast agent is a gadolinium-based contrast agent, and the $T_2$ contrast agent is an iron-based contrast agent. The $T_1$ contrast agent used in clinic is mainly a gadolinium-based chelate, which is a micromolecule drug, comprising Magnevist®, Gadavist®, Dotarem®, Omniscan®, Vasovist®, and the like. American Food and Drug Administration (FDA) warned that the gadolinium-based contrast agent for clinical use has brain deposition and nephrotoxicity, so that there are certain risks in clinical application.

Iron is one of essential elements for human body, and ferroferric oxide has excellent biocompatibility. Compared with the gadolinium-based chelate, the ferroferric oxide is safer and more reliable when used as the MRI contrast agent for clinical diagnosis. However, the ferroferric oxide mainly exists as the $T_2$ contrast agent, and its characteristic of generating the dark signal seriously affects the clinical application. An $r_2$ value of the ferroferric oxide is positively correlated with a particle size. With the increase of the particle size, the saturation magnetization ($M_z$) of the ferroferric oxide is also increased, and the $r_2$ value is also increased. However, the saturation magnetization of the ferroferric oxide (magnetic the ferroferric oxide nanoparticle (ES-MION)) with a particle size of 5 nm or less is obviously reduced, and the $r_2$ value is lower, so that the ferroferric oxide may be used as the $T_1$ contrast agent. Therefore, the magnetic ferroferric oxide nanoparticle with higher $r_1$ value and lower $r_2$ value and the particle size less than 5 nm is expected to become a safe and reliable $T_1$ contrast agent with good imaging effect to be applied to clinical diagnosis. In the related art, the ferroferric oxide with the particle size of 5 nm or less is prepared from polymers such as polyvinyl alcohol (PVA ), which shows the characteristics of the $T_1$ contrast agent, but the dispersibility is poor and the imaging effect is limited.

**SUMMARY**

The present disclosure aims to solve at least one of the technical problems existing in the prior art. Therefore, the present disclosure provides a magnetic ferroferric oxide nanoparticle, which has a higher $r_1$ value and a lower $r_2/r_1$ ratio, good water solubility, high stability and good biocompatibility.

Meanwhile, the present disclosure further provides a preparation method for the magnetic ferroferric oxide nanoparticle and use of the magnetic ferroferric oxide nanoparticle.

Specifically, technical solutions used in the present disclosure are specifically as follows.

In a first aspect, the present disclosure provides a magnetic ferroferric oxide nanoparticle, wherein the magnetic ferroferric oxide nanoparticle comprises ferroferric oxide and a hydrophilic macromolecule, and the ferroferric oxide and the hydrophilic macromolecule are in at least one of the following relationships (1) and (2): (1) the hydrophilic macromolecule is adsorbed on a surface of the ferroferric oxide; and (2) the ferroferric oxide and the hydrophilic macromolecule are mutually embedded or occluded. The magnetic ferroferric oxide nanoparticle simultaneously has the following properties 1) to 4):

1) an average particle size is greater than 2 nm and less than 5 nm;

2) an electrokinetic potential is less than or equal to -10 mV;

3) a hydrodynamic diameter is less than or equal to 20 nm; and

4) a longitudinal magnetic relaxation rate $r_1$ value under a magnetic field intensity of 3.0 T is greater than 5 mM$^{-1}$ s$^{-1}$; and a longitudinal magnetic relaxation rate $r_1$ value under a magnetic field intensity of 1.0 T is greater than 10 mM$^{-1}$ s$^{-1}$.

The magnetic ferroferric oxide nanoparticle according to the present disclosure in the first aspect comprises at least the following beneficial effects.

In the present disclosure, the hydrophilic macromolecule is used as a stabilizer, and forms the magnetic ferroferric oxide nanoparticle (which may be synthesized by coprecipitation) with the ferroferric oxide by surface adsorption and mutual embedding or occlusion. The magnetic ferroferric oxide nanoparticle is a water-soluble nano-drug, and compared with the ferroferric oxides obtained by surface modification of existing ferroferric oxide with a macromolecule and the ferroferric oxides synthesized by an oil-phase method in the related art, the magnetic ferroferric oxide nanoparticle of the present disclosure has better water solubility and higher stability, can increase the $r_1$ value and reduce the $r_2/r_1$ ratio, and may be used as a safe and reliable $T_1$ contrast agent.

In some embodiments of the present disclosure, a Zeta potential of the magnetic ferroferric oxide nanoparticle is less than or equal to -30 mV; preferably, the Zeta potential is less than or equal to -35 mV; and more preferably, the Zeta potential is -60 mV to -35 mV.

In some embodiments of the present disclosure, a hydrodynamic diameter of the magnetic ferroferric oxide nanoparticle is less than or equal to 20 nm; and preferably, the hydrodynamic diameter is less than or equal to 15 nm.

In some embodiments of the present disclosure, the hydrophilic macromolecule comprises any one or a copolymer or mixture of several of a carboxylic acid-containing macromolecule, an amino-containing macromolecule, a hydroxyl-containing macromolecule, an amide-containing macromolecule and a polysaccharide.

In some embodiments of the present disclosure, a molecular weight of the hydrophilic macromolecules is 1,000 to 10,000, and a suitable macromolecule may be selected according to a specific condition.

In some embodiments of the present disclosure, the carboxylic acid-containing macromolecule comprises any one or more of polyglutamic acid, polyaspartic acid, polymaleic acid, poly(2-ethylacrylic acid) and polyepoxysuccinic acid.

In some embodiments of the present disclosure, the amino-containing macromolecule comprises any one or more of polylysine, polyhistidine, poly-L-arginine and polydimethyl diallyl ammonium chloride.

In some embodiments of the present disclosure, the hydroxyl-containing macromolecule comprises any one or more of polyserine, polythreonine, polytyrosine and tannic acid.

In some embodiments of the present disclosure, the amide-containing macromolecule comprises any one or more of polyglutamine, polyasparamide, polyacrylamide and polymethacrylamide.

In some embodiments of the present disclosure, the polysaccharide comprises one or two of hyaluronic acid and sodium alginate.

In a second aspect, the present disclosure provides a preparation method for the magnetic ferroferric oxide nanoparticle as described above, which comprises a step of:
coprecipitating ferrous ions and ferric ions to form the magnetic ferroferric oxide nanoparticle by using the hydrophilic macromolecule as a stabilizer.

After coprecipitation, the ferrous ions and the ferric ions react to form the ferroferric oxide, and the ferroferric oxide and the hydrophilic macromolecule form the magnetic ferroferric oxide nanoparticle by surface adsorption and mutual embedding or occlusion.

More specifically, the hydrophilic macromolecule is used as the stabilizer, so that the ferrous ions and the ferric ions are subjected to the coordination reaction with the hydrophilic macromolecule first, and then the alkali liquor is added for the coprecipitation reaction to obtain the magnetic ferroferric oxide nanoparticle.

In some embodiments of the present disclosure, the preparation method for the magnetic ferroferric oxide nanoparticle comprises the following steps of:
heating a hydrophilic macromolecule solution, then mixing the hydrophilic macromolecule solution after being subjected to heating with an iron ion mixed solution comprising ferrous ions and ferric ions for a coordination reaction, and then adding an alkali liquor for a coprecipitation reaction to obtain the magnetic ferroferric oxide nanoparticle.

In some embodiments of the present disclosure, in the iron ion mixed solution, a concentration of ferrous ions is 30 mM to 500 mM, preferably 50 mM to 250 mM, more preferably 150 mM to 250 mM, and further preferably 200 mM to 250 mM; and a concentration of ferric ions is 60 mM to 1,000 mM, preferably 100 mM to 500 mM, more preferably 300 mM to 500 mM, and further preferably 350 mM to 500 mM.

In some embodiments of the present disclosure, a concentration of the hydrophilic macromolecule solution is 0.1 mg/mL to 20 mg/mL, and preferably 0.5 mg/mL to 5 mg/mL.

In some embodiments of the present disclosure, a volume ratio of the hydrophilic macromolecule solution to the iron ion mixed solution is (1 to 500): 1, and preferably (10 to 50): 1.

In some embodiments of the present disclosure, the hydrophilic macromolecule solution and the iron ion mixed solution are both an aqueous solution.

In some embodiments of the present disclosure, the ferrous ions are obtained by hydrolysis of water-soluble ferrous salt, and the water-soluble ferrous salt comprises any one or more of ferrous chloride, ferrous nitrate, ferrous bromide and ferrous sulfate, and preferably the ferrous sulfate.

In some embodiments of the present disclosure, the ferric ions are obtained by hydrolysis of water-soluble ferric salt, and the water-soluble ferric salt comprises any one or more of ferric chloride, ferric nitrate, ferric bromide and ferric sulfate, and preferably the ferric chloride.

In some embodiments of the present disclosure, a pH value of the alkali liquor is 10 to 12, and preferably 11.5. After the alkali liquor is added into a mixed solution of the iron ion mixed solution and the hydrophilic macromolecule solution, a pH value of a reaction system is 8 to 10, and preferably 9.5.

In some embodiments of the present disclosure, the alkali liquor comprises at least one of sodium hydroxide and an aqueous solution thereof, potassium hydroxide and an aqueous solution thereof, and ammonia water, and preferably the ammonia water. Preferably, a mass concentration of the ammonia water is 0.5% to 5%, and preferably 1% to 4%.

In some embodiments of the present disclosure, the alkali liquor is in a form of solution, and a volume ratio of the alkali liquor to the hydrophilic macromolecule solution is 1: (2 to 10), and preferably 1: (4 to 6).

In some embodiments of the present disclosure, independently, the hydrophilic macromolecule solution is heated at a temperature of 25 °C to 100 °C, preferably 100 °C; and the coprecipitation reaction is conducted at a temperature of 25 °C to 100 °C, and preferably 100 °C.In some embodiments of the present disclosure, the coprecipitation reaction lasts for more than 5 minutes, and preferably 40 minutes to 100 minutes.

In a third aspect, the present disclosure provides use of the magnetic ferroferric oxide nanoparticle as described above in preparing a magnetic resonance imaging contrast agent.

In some embodiments of the present disclosure, the MRI contrast agent is a longitudinal relaxation contrast agent ($T_1$ contrast agent).

Compared with the prior art, the present disclosure has the following beneficial effects.

The magnetic ferroferric oxide nanoparticle of the present disclosure has a higher $r_1$ value (>5 mM$^{-1}$ s$^{-1}$, 3.0 T; and >10 mM$^{-1}$ s$^{-1}$, 1.0 T), and a lower $r_2/r_1$ ratio (<13.0, 3.0 T), and may be compared with a gadolinium-based chelate contrast agent for clinical use. As an iron-based MRI contrast agent, the magnetic ferroferric oxide nanoparticle of the present disclosure is synthesized by the coprecipitation method with the hydrophilic macromolecule as the stabilizer, has a good imaging effect, a low signal-to-noise ratio, good water solubility, good stability and a small hydrodynamic diameter, and is beneficial for blood circulation. Moreover, the hydrophilic macromolecule used in the present disclosure is biodegradable, and has better biocompatibility and higher safety compared with the gadolinium-based chelate contrast agent for clinical use.

## BRIEF DESCRIPTION OF THE DRAWINGS

FIG. 1 shows a transmission electron micrograph and a particle size distribution diagram of γ-PGA-ES-MION6 in Example 1;

FIG. 2 shows a hydraulic diameter and a Zeta potential diagram of γ-PGA-ES-MION6 in Example 1;

FIG. 3 shows MRI relaxation rate of γ-PGA-ES-MION6 in Example 1;

FIG. 4 shows a saturation magnetization diagram of γ-PGA-ES-MION6 in Example 1;

FIG. 5 shows an X-ray photoelectron spectrogram and an X-ray diffraction diagram of γ-PGA-ES-MION6 in Example 1;

FIG. 6 shows an infrared absorption spectrogram of γ-PGA-ES-MION6 in Example 1;

FIG. 7 shows $T_1$-weighted MRI images of γ-PGA-ES-MION6 in Example 1 and pure water and corresponding MRI signal intensities;

FIG. 8 is a MRI image showing a tumor-bearing mouse after being injected with γ-PGA-ES-MION6 in Example 1;

FIG. 9 shows a transmission electron micrograph of PASP-ES-MION9 in Example 2;

FIG. 10 shows a hydraulic diameter and a Zeta potential diagram of PASP-ES-MION9 in Example 2;

FIG. 11 shows MRI relaxation rate of PASP-ES-MION9 in Example 2;

FIG. 12 shows an X-ray diffraction diagram and an X-ray photoelectron spectrogram of PASP-ES-MION9 in Example 2;

FIG. 13 shows a $T_1$-weighted MRI image of PASP-ES-MION9 in Example 2 and a corresponding MRI signal intensity;

FIG. 14 shows an infrared absorption spectrogram of PASP-ES-MION9 in Example 2;

FIG. 15 a MRI image showing a tumor-bearing mouse after being injected with PASP-ES-MION9 in Example 2;

FIG. 16 shows MRI relaxation rate of HPMA-ES-MION in Example 3;

FIG. 17 shows MRI relaxation rate of PEAA-ES-MION in Example 4;

FIG. 18 shows MRI relaxation rate of PESA-ES-MION in Example 5;

FIG. 19 shows MRI relaxation rate of ε-PL-ES-MION in Example 6;

FIG. 20 shows MRI relaxation rate of PLH-ES-MION in Example 7;

FIG. 21 shows MRI relaxation rate of PLR-ES-MION in Example 8;

FIG. 22 shows MRI relaxation rate of PDDA-ES-MION in Example 9;

FIG. 23 shows MRI relaxation rate of PSer-ES-MION in Example 10;

FIG. 24 shows MRI relaxation rate of PThr-ES-MION in Example 11;

FIG. 25 shows MRI relaxation rate of PTyr-ES-MION in Example 12;

FIG. 26 shows MRI relaxation rate of TA-ES-MION in Example 13;

FIG. 27 shows MRI relaxation rate of PolyQ-ES-MION in Example 14;

FIG. 28 shows MRI relaxation rate of PHEA-ES-MION in Example 15;

FIG. 29 shows MRI relaxation rate of PAM-ES-MION in Example 16;

FIG. 30 shows MRI relaxation rate of PMAM-ES-MION in Example 17;

FIG. 31 shows MRI relaxation rate of HA-ES-MION in Example 18;

FIG. 32 shows MRI relaxation rate of SA-ES-MION in Example 19;

FIG. 33 shows MRI relaxation rate of γ-PGA/PASP-ES-MION in Example 20; and

FIG. 34 shows MRI relaxation rate of HPMA/PASP-ES-MION in Example 21.

## DETAILED DESCRIPTION

Technical solutions of the present disclosure are further described in detail hereinafter with reference to specific examples. Raw materials used in the following examples may be commercially available conventionally, unless otherwise specified; and the processes are all conventional processes in the art, unless otherwise specified.

**Example 1**

Preparation of magnetic ferroferric oxide nanoparticle ($\gamma$-PGA-ES-MION):

20 mL of polyglutamic acid $\gamma$-PGA (Mw = 2,000) aqueous solution was subjected to bubble deoxygenation with nitrogen in a three-necked flask for 1 hour, and then heated at 100°C to reflux. 0.4 mL of a mixed aqueous solution containing $FeSO_4$ and $FeCl_3$ was added into the flask, and then added with 6 mL of ammonia water. The mixture reacted for 1 hour under magnetic stirring at 100°C, and was allowed to stand and cooled. The sample after the reaction was dialyzed and purified, and the final sample was marked as $\gamma$-PGA-ES-MION. Concentrations of the polyglutamic acid aqueous solution, the mixed aqueous solution and the ammonia water and corresponding samples were shown in Table 1.

Physical properties of the sample in Example 1 were characterized. A recovery rate of iron of the sample in Example 1 was calculated to be 96%, which indicated that a utilization rate of a raw material was high, so that a cost could be effectively reduced. The sample in Example 1 and commercially available products Gadavist and Magnevist were respectively prepared into 6 aqueous solutions with different concentrations, and in-vitro imaging tests were carried out by 1.0 T, clinical 3.0 T and 7.0 T MRI systems to obtain longitudinal relaxation time $T_1$ and transverse relaxation time $T_2$. A longitudinal magnetic relaxation rate $r_1$ and a transverse magnetic relaxation rate $r_2$ were calculated by the following formula (c is a concentration of a magnetic substance in a contrast agent, and $T_i$ is relaxation time, wherein i = 1 or 2), and results were shown in Table 1.

$$r_i = \frac{\Delta(1/T_i)}{\Delta c}$$

Table 1 Synthesis of $\gamma$-PGA-ES-MION and MRI characterization results

| Name of sample | $C_{\gamma\text{-PGA}}$ (mg/mL)-a | $C_{FeCl3}$ (mM)[a] | $C_{FeSO4}$ (mM)[a] | $C_{NH3\cdot H2O}$ (%)[a] | Recovery rate of iron (%)[b] | $H_0$ (T) | $r_1$ (mM$^{-1}$s$^{-1}$) | $r_2$ (mM$^{-1}$s$^{-1}$) | $r_2/r_1$ |
|---|---|---|---|---|---|---|---|---|---|
| $\gamma$-PGA-ES-MION1 | 2.0 | 500 | 250 | 28 | 98.0 | 3.0 | 8.59 | 268.9 | 31.3 |
| $\gamma$-PGA-ES-MION2 | 1.0 | 500 | 250 | 28 | 93.8 | 3.0 | 8.41 | 285.9 | 34.0 |
| $\gamma$-PGA-ES-MION3 | 0.5 | 500 | 250 | 28 | 78.0 | 3.0 | 7.04 | 221.7 | 31.5 |
| $\gamma$-PGA-ES-MION4 | 0.25 | 500 | 250 | 28 | 25.4 | 3.0 | 7.26 | 247.9 | 34.2 |
| $\gamma$-PGA-ES-MION5 | 2.0 | 500 | 250 | 0.5 | 92.1 | 3.0 | 2.79±0.06 | 47.5±0.2 | 17.0±0.3 |
| | | | | | | 7.0 | 1.47±0.01 | 71.0±4.2 | 48.3±3.1 |
| $\gamma$-PGA-ES-MION6 | 2.0 | 500 | 250 | 1 | 96.1 | 3.0 | 5.70±0.04 | 70.2±1.5 | 12.3±0.2 |
| | | | | | | 1.0 | 10.11±0.69 | 103.6±4.5 | 10.3±1.1 |
| $\gamma$-PGA-ES-MION7 | 2.0 | 500 | 250 | 2 | 95.3 | 3.0 | 6.42±0.03 | 104.3±0.4 | 16.3±0.1 |
| $\gamma$-PGA-ES-MION8 | 2.0 | 500 | 250 | 4 | 98.7 | 3.0 | 6.94±0.04 | 119.4±2.8 | 17.2±0.4 |
| Gadavist | - | - | - | | - | 3.0 | 4.64 | 4.81 | 1.04 |
| Magnevist | - | - | - | | - | 3.0 | 4.58 | 4.98 | 1.09 |

[a] is a concentration of a reactant added before the reaction; and
[b] is a molar percentage of an iron content of the ferroferric oxide in the raw material iron added.

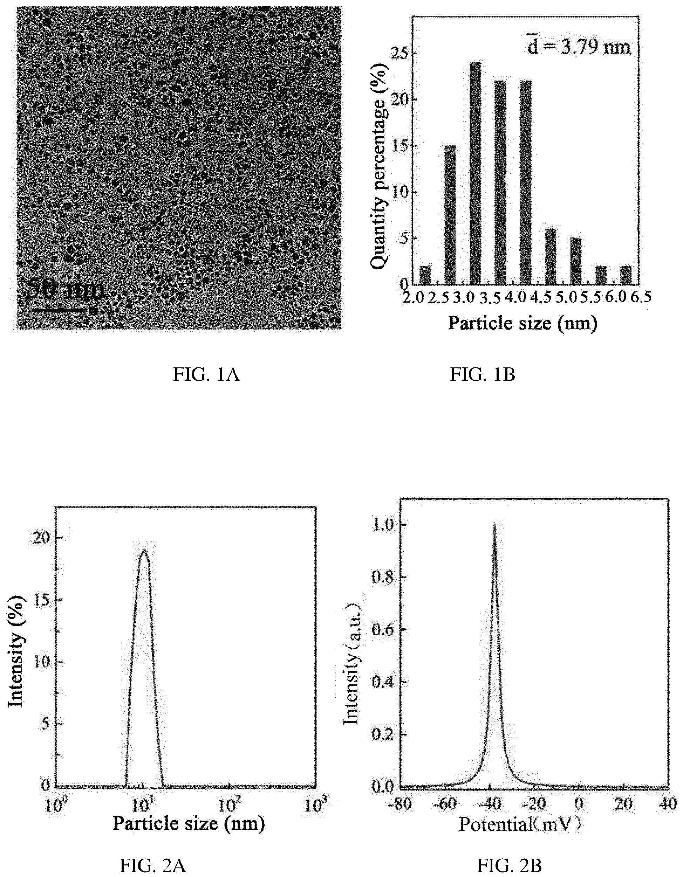

Specifically, FIG. 1 shows a transmission electron micrograph (TEM) and a particle size distribution diagram of the sample $\gamma$-PGA-ES-MION6 in Example 1. It can be seen from the transmission electron micrograph of FIG. 1 (a) that the nanoparticles are uniform in size and evenly dispersed, and particle size distributions of 100 nanoparticles randomly selected from FIG. 1 (a) are counted to obtain the distribution diagram of FIG. 1 (b). It can be seen from the distribution diagram that the particle size of the sample is mainly about 3.5 nm. An average particle size of the sample $\gamma$-PGA-ES-MION6 is less than 5 nm, and the sample has the potential to be the $T_1$ contrast agent from the view of particle size. FIG. 2 shows a hydraulic diameter and a Zeta potential diagram of the sample $\gamma$-PGA-ES-MION6 in Example 1. A hydraulic diameter of the sample is 8.7 nm, and a polymer dispersity index (PDI) of the sample is 0.265, which indicates good dispersity. A Zeta potential of the sample is -37.3 mV, and water phase dispersity of the sample can be improved by charge repulsion, which is beneficial for blood circulation of the sample.

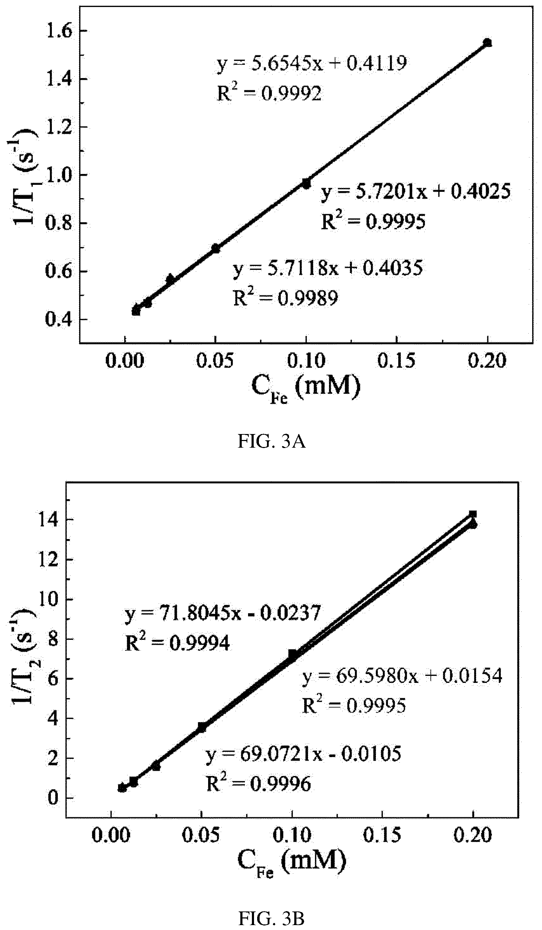

The concentrations of the polyglutamic acid aqueous solution, the mixed aqueous solution and the ammonia water have a great influence on the MRI performance of the sample. Under different synthetic conditions, the sample $\gamma$-PGA-ES-MION6 has the best comprehensive MRI performance. FIG. 3 shows MRI relaxation rate of the sample $\gamma$-PGA-ES-MION6 in Example 1 (three samples $\gamma$-PGA-ES-MION6-1, $\gamma$-PGA-ES-MION6-2 and $\gamma$-PGA-ES-MION6-3 were tested in parallel for three times). A slope of a fitted line is a corresponding magnetic relaxation rate. It can be seen from FIG. 3 (a) or (b) that goodness of linear fitting is 0.999 or more, which proves that the sample has an excellent linear relationship, thus indicating that the relaxation time may be changed linearly with a change of a concentration of the sample.

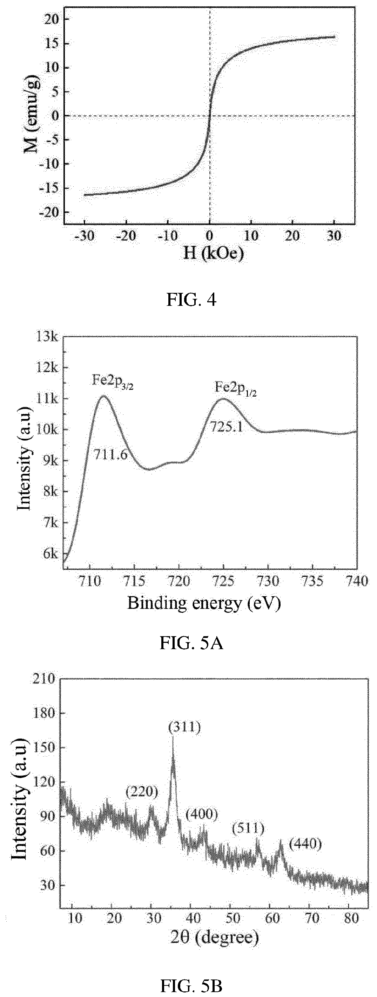

Table 1 shows the $r_1$ value and the $r_2/r_1$ ratio of the sample $\gamma$-PGA-ES-MION1-8 in Example 1 under different magnetic field intensities (7.0 T, 3.0 T or 1.0 T), and simultaneously shows the $r_1$ values and the $r_2/r_1$ ratios of the commercially available products Gadavist and Magnevist under the magnetic field intensity of 3.0 T. The $r_1$ value of the sample $\gamma$-PGA-ES-MION6 in Example 1 under the magnetic field intensity of 3.0 T is 5.7 mM$^{-1}$ s$^{-1}$, which is higher than the $r_1$ value of 4.6 mM$^{-1}$ s$^{-1}$ of the commercially available products, thus indicating an excellent magnetic resonance imaging capability. FIG. 4 shows a saturation magnetization diagram of $\gamma$-PGA-ES-MION6 in Example 1. It can be seen from the figure that the sample in Example 1 is paramagnetic, which accords with the property of the magnetic ferroferric oxide nanoparticle. A magnitude of the saturation magnetization is positively correlated with the particle size. It can be seen from the figure that the saturation magnetization of the sample in Example 1 is 16 emu/g, which corresponds to the particle size less than 5 nm shown in the electron micrograph in FIG. 1. The $r_2$ value of the contrast agent is positively correlated with the saturation magnetization, and small saturation magnetization corresponds to a small $r_2$ value, which corresponds to the small $r_2$ value shown in Table 1.

FIG. 5 shows an X-ray photoelectron spectrogram (XPS) and an X-ray diffraction diagram (XRD) of $\gamma$-PGA-ES-MION6 in Example 1. Peaks centered at 711.6 eV and 725.1 eV in the XPS of FIG. 5 (a) correspond to binding energies of $Fe^{2+}2p$ and $Fe^{3+}2p$ respectively, which indicates that trivalent iron and divalent iron exist at the same time, and proves that the sample in Example 1 is the ferroferric oxide. Two characteristic peaks ($2\theta \approx 35.6°$ and $2\theta \approx 62.9°$) in the XRD of FIG. 5 (b) correspond to crystal layers [(311) and (440)] of the ferroferric oxide, which proves that the sample in Example 1 is the ferroferric oxide in a crystal structure. The XRD and the XPS jointly prove the successful preparation of the ferroferric oxide in Example 1, and then prove the successful preparation of the magnetic ferroferric oxide nanoparticle in Example 1 in combination with the electron micrograph in FIG. 1.

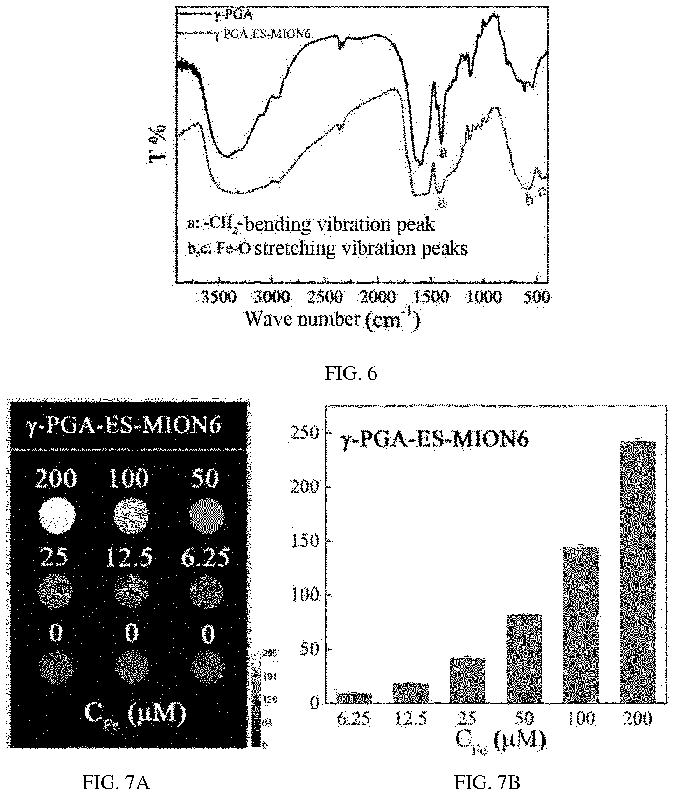

FIG. 6 shows an infrared absorption spectrogram of $\gamma$-PGA-ES-MION6 prepared in Example 1. An infrared absorption curve of $3\gamma$-PGA and an infrared absorption curve of $\gamma$-PGA-ES-MION6 both show an absorption peak a (1,404 cm$^{-1}$), which is a bending vibration peak of $-CH_2-$, thus indicating the existence of the $\gamma$-PGA in the sample. In addition, the infrared absorption curve of the $\gamma$-PGA-ES-MION6 shows absorption peaks b and c, which are stretching vibration peaks of Fe-O, while the infrared absorption curve of the $\gamma$-PGA does not show the absorption peaks b and c, which further proves the successful preparation of the magnetic ferroferric oxide nanoparticle $\gamma$-PGA-ES-MION6.

FIG. 7 shows $T_1$-weighted MRI images of $\gamma$-PGA-ES-MION6 aqueous solutions at different concentrations in Example 1 and corresponding MRI signal intensities. The magnetic field intensity is 3.0 T, and scanning parameters are: TE = 8.6 ms and TR = 500 ms. It can be seen from the MRI image of FIG. 7(a) that an MRI signal of the aqueous solution containing the sample $\gamma$-PGA-ES-MION6 is obviously enhanced compared with pure water (a concentration of iron is 0), and shows a gradient enhancement with the increase of the concentration of the sample. It can be seen from FIG. 7(b) that, when the concentration of iron in the $\gamma$-PGA-ES-MION6 solution is 200 $\mu$M, a signal-to-noise ratio ($\triangle$SNR) of the MRI is 240%, which indicates that the sample $\gamma$-PGA-ES-MION6 can effectively improve the contrast and sensitivity of the MRI, thus showing the good magnetic resonance imaging performance of the $\gamma$-PGA-ES-MION6 in Example 1.

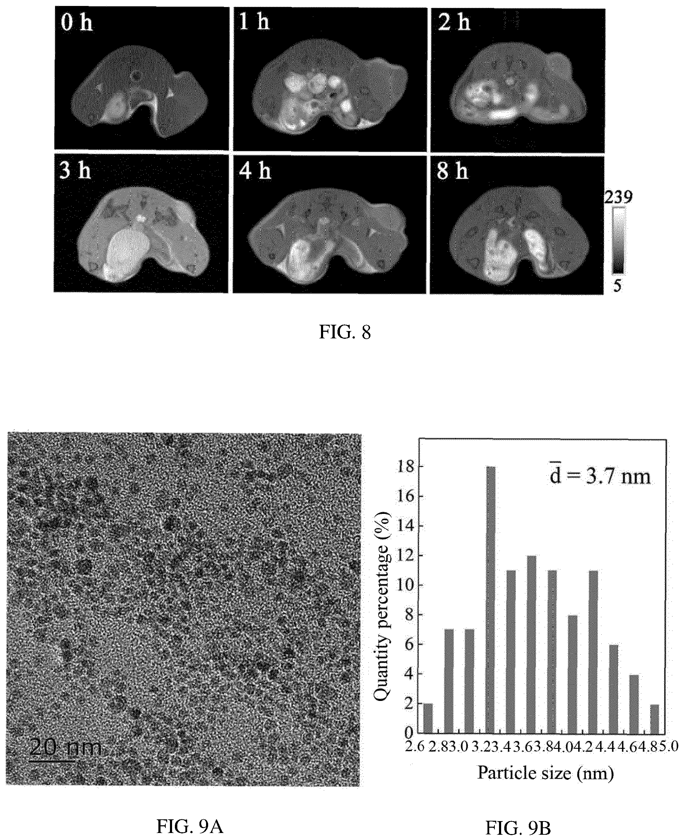

FIG. 8 is a MRI image showing a tumor-bearing mouse after being injected with $\gamma$-PGA-ES-MION6 in Example 1, with an injection dosage of 5 mg/kg. 7.0 T magnetic resonance imaging (MRI) is carried out on the mouse at 0 hour, 1 hour, 2 hours, 3 hours, 4 hours and 8 hours before and after tail vein injection of the sample into the mouse. It can be seen from the figure that, with the increase of injection time of the sample, an MRI signal of a tumor shows a trend of increasing first and then decreasing, and the MRI signal is the highest at 3 hours after injection. In-vivo imaging results show that the sample $\gamma$-PGA-ES-MION6 in Example 1 has a good magnetic resonance imaging effect on the tumor of the mouse.

## Example 2

Preparation of magnetic ferroferric oxide nanoparticle (PASP-ES-MION):

20 mL of polyaspartic acid PASP ($M_w$ = 7,000 to 8,000) aqueous solution was subjected to bubble deoxygenation with nitrogen in a three-necked flask for 1 hour, and then heated at 100°C to reflux. 0.4 mL of a mixed aqueous solution containing $FeSO_4$ and $FeCl_3$ was added into the flask, and then added with 6 mL of ammonia water. The mixture reacted for 1 hour under magnetic stirring at 100°C, and was allowed to stand and cooled. The sample after the reaction was dialyzed and purified, and the final sample was marked as PASP-ES-MION. Concentrations of the polyaspartic acid aqueous solution, the mixed aqueous solution and the ammonia water and corresponding samples were shown in Table 2.

Physical properties of the sample in Example 2 were characterized. A recovery rate of iron of the sample in Example 2 was calculated to be 89.6%, which indicated that a utilization rate of a raw material was high, so that cost saving was realized. The sample in Example 2 was prepared into 6 aqueous solutions with different concentrations, and in-vitro imaging tests were carried out by a clinical 3.0 T MRI system to obtain longitudinal relaxation time $T_1$ and transverse relaxation time $T_2$. A longitudinal magnetic relaxation rate $r_1$ and a transverse magnetic relaxation rate $r_2$ were calculated, and results were shown in Table 2.

Table 2 Synthesis of PASP-ES-MION and MRI characterization results

| Name of sample | $C_{PASP}$ (mg/mL)-a | $C_{FeCl3}$ (mM)[a] | $C_{FeSO4}$ (mM)[a] | $C_{NH3 \cdot H2O}$ (%)a | Recovery rate of iron (%)[b] | $H_0$ (T) | $r_1$ (mM$^{-1}$ s$^{-1}$) | $r_2$ (mM$^{-1}$ s$^{-1}$) | $r_2/r_1$ |
|---|---|---|---|---|---|---|---|---|---|
| PASP-ES-MION1 | 4.0 | 500 | 250 | 1 | 92.7 | 3.0 | 0.99 | 3.71 | 3.76 |
| PASP-ES-MION2 | 2.0 | 500 | 250 | 1 | 96.6 | 3.0 | 2.01 | 14.04 | 6.98 |
| PASP-ES-MION3 | 1 | 500 | 250 | 1 | 89.3 | 3.0 | 4.66 | 88.50 | 19.00 |
| PASP-ES-MION4 | 0.5 | 500 | 250 | 1 | 90.3 | 3.0 | 5.41 | 153.09 | 28.31 |
| PASP-ES-MION5 | 2 | 500 | 250 | 8 | 99.2 | 3.0 | 5.53±0.04 | 31.0±0.05 | 5.62±0.1 |
| PASP-ES-MION6 | 2 | 500 | 250 | 4 | 98.4 | 3.0 | 5.22±0.03 | 27.3±0.56 | 5.22±0.1 |
| PASP-ES-MION7 | 2 | 500 | 250 | 2 | 98.8 | 3.0 | 3.83±0.02 | 25.1±0.51 | 6.54±0.2 |
| PASP-ES-MION8 | 2 | 500 | 250 | 0.5 | 97.5 | 3.0 | 0.32±0.01 | 5.8±0.08 | 18.2±0.4 |
| PASP-ES-MION9 | 1.5 | 375 | 187.5 | 4 | 89.6 | 3.0 | 7.3 | 35.8 | 4.9 |
| PASP-ES-MION10 | 1 | 250 | 125 | 4 | 87.7 | 3.0 | 6.2 | 33.0 | 5.3 |
| PASP-ES-MION11 | 0.5 | 125 | 62.5 | 4 | 97.7 | 3.0 | 6.0 | 38.8 | 6.5 |

[a] is a concentration of a reactant added before the reaction; and
[b] is a molar percentage of an iron content of the ferroferric oxide in the raw material iron added.

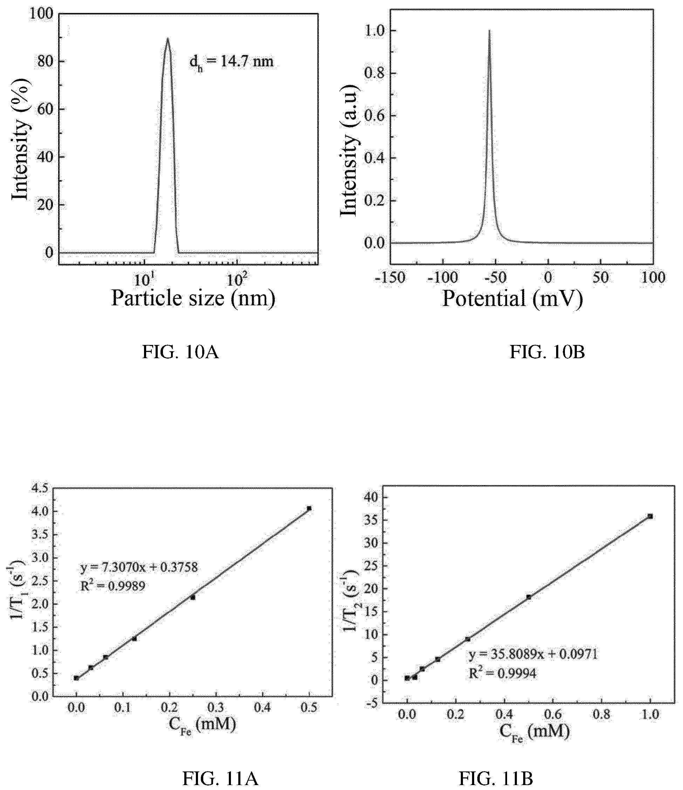

FIG. 9 shows a transmission electron micrograph and a particle size distribution diagram of the sample PASP-ES-MION9 in Example 2. It can be seen from the transmission electron micrograph of FIG. 9 (a) that the nanoparticles are uniform in size and evenly dispersed, and particle size distributions of 100 nanoparticles randomly selected from FIG. 9 (a) are counted to obtain the distribution diagram of FIG. 9 (b). It can be seen from the distribution diagram that the particle size of the sample mainly ranges from 2.6 nm to 5.0 nm, and an average particle size of the sample is 3.7 nm, thus meeting the requirement that the particle size of the ferroferric oxide as the $T_1$ contrast agent is less than 5 nm. FIG. 10 shows a hydraulic diameter and a Zeta potential diagram of the sample PASP-ES-MION9 in Example 2. It is measured that an average value of hydraulic diameters of three samples (PASP-ES-MION9-1, PASP-ES-MION9-2 and PASP-ES-MION9-3) is 14.7 nm, and a PDI of the sample is 0.224, which indicates good dispersity. A Zeta potential of the sample is -50.7 mV, and water phase dispersity of the sample can be improved by charge repulsion, which is beneficial for blood circulation of the sample.

The concentrations of the polyaspartic acid aqueous solution, the mixed aqueous solution and the ammonia water have a great influence on the MRI performance of the sample. Under different synthetic conditions, the sample PASP-ES-MION9-10 has a better comprehensive MRI performance. Taking the PASP-ES-MION9 as an example, FIG. 11 shows MRI relaxation rate of the sample PASP-ES-MION9 in Example 2. It can be seen from FIG. 11 (a) or FIG. (b) that goodness of linear fitting of a fitted line is 0.99 or more, which proves that the sample has a good linear relationship. Table 2 shows the $r_1$ value and the $r_2/r_1$ ratio of the sample PASP-ES-MION9 in Example 2 under the magnetic field intensity of 3.0 T. The $r_1$ value of the sample PASP-ES-MION9 in Example 2 under the magnetic field intensity of 3.0 T is 7.3 mM$^{-1}$ s$^{-1}$, which is higher than the $r_1$ value of 4.64 mM$^{-1}$ s$^{-1}$ of the commercially available products. In addition, the $r_2/r_1$ ratio of the sample PASP-ES-MION9 under the magnetic field intensity of 3.0 T is only 4.9. The results show that the sample PASP-ES-MION9 in Example 2 under the magnetic field intensity of 3.0 T has an extremely high $r_1$ value and an extremely low $r_2/r_1$ ratio.

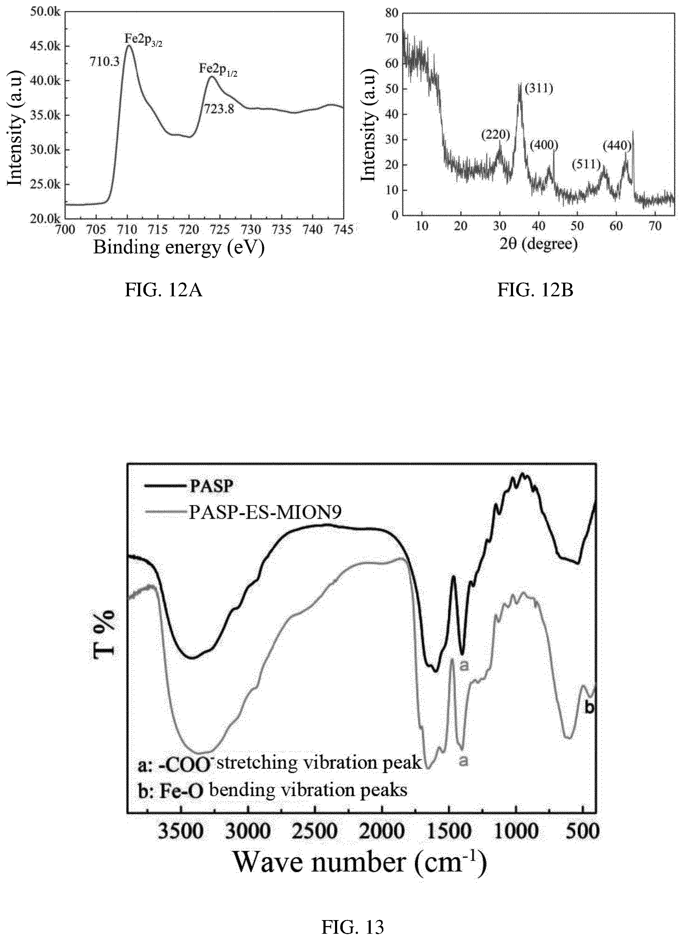

FIG. 12 shows an X-ray photoelectron spectrogram and an X-ray diffraction diagram of the PASP-ES-MION9 in Example 2. Peaks centered at 710.3 eV and 723.8 eV in the XPS of FIG. 12 (a) correspond to binding energies of $Fe^{2+}2p$ and $Fe^{3+}2p$ respectively, which indicates that trivalent iron and divalent iron exist at the same time, and proves that the sample in Example 2 is the ferroferric oxide. Two characteristic peaks ($2\theta \approx 35.3°$ and $2\theta \approx 62.4°$) in the XRD of FIG. 12 (b) correspond to crystal layers [(311) and (440)] of the ferroferric oxide, which proves that the sample in Example 2 is the ferroferric oxide in a crystal structure. The XRD and the XPS jointly prove the successful preparation of the ferroferric oxide in Example 2, and then prove the successful preparation of the magnetic ferroferric oxide nanoparticle in Example 2 in combination with the electron micrograph in FIG. 9.

FIG. 13 shows an infrared absorption spectrogram of PASP-ES-MION9 prepared in Example 2. An infrared absorption curve of PASP and an infrared absorption curve of PASP-ES-MION9 both show an absorption peak a (1,400 cm$^{-1}$), which is a stretching vibration peak of -COO$^-$, thus indicating the existence of the PASP in the sample. In addition, the infrared absorption curve of PASP-ES-MION9 shows an absorption peak b, which is a stretching vibration peak of Fe-O, while the infrared absorption curve of the PASP does not show the absorption peak b, which further proves the successful preparation of the magnetic ferroferric oxide nanoparticle PASP-ES-MION9.

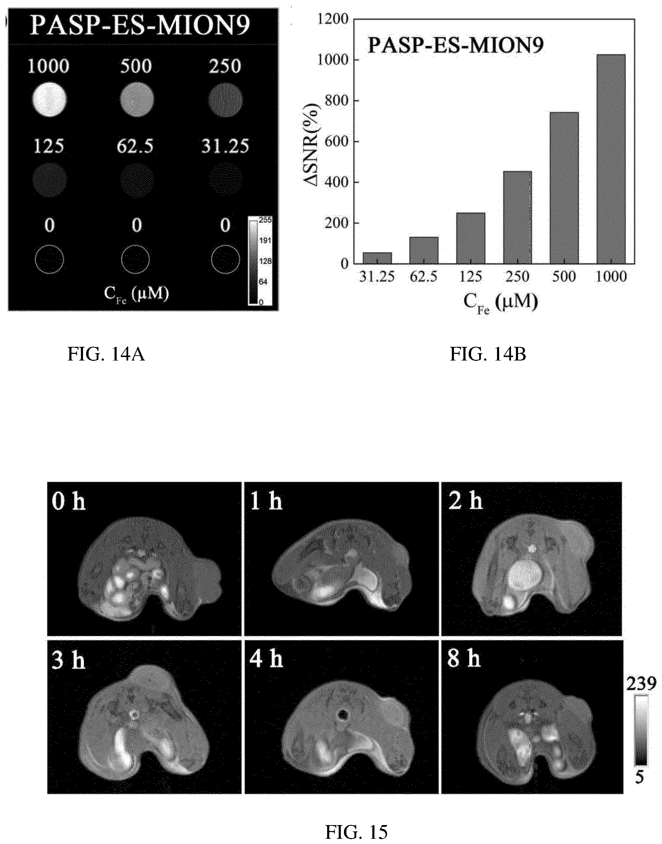

FIG. 14 shows $T_1$-weighted MRI images of PASP-ES-MION9 aqueous solutions at different concentrations in Example 2 and corresponding MRI signal intensities. The magnetic field intensity is 3.0 T, and scanning parameters are: TE = 8.4 ms and TR = 200 ms. It can be seen from the MRI image of FIG. 14 (a) that an MRI signal of the aqueous solution containing the sample PASP-ES-MION9 is obviously enhanced compared with pure water (a concentration of iron is 0), and shows a gradient enhancement with the increase of the concentration of the sample. It can be seen from FIG. 14 (b) that, when the concentration of iron in the sample PASP-ES-MION9 aqueous solution is 1,000 $\mu$M, $\triangle$SNR of the MRI is 1,025 %, which proves that the PASP-ES-MION9 can effectively improve the contrast and sensitivity of the MRI, thus showing the excellent magnetic resonance imaging performance of the PASP-ES-MION9 in Example 2.

FIG. 15 shows a MRI image showing a tumor-bearing mouse after being injected with PASP-ES-MION9 in Example 2, with an injection dosage of 5 mg/kg. 7.0 T magnetic resonance imaging (MRI) is carried out on the mouse at 0 hour, 1 hour, 2 hours, 3 hours, 4 hours and 8 hours before and after tail vein injection of the sample into the mouse. It can be seen from the figure that, with the increase of injection time of the sample, an MRI signal of a tumor is increased first and then decreased, and the MRI signal is the highest at 3 hours after injection. In-vivo imaging results show that the PASP-ES-MION9 in Example 2 has a good magnetic resonance imaging effect on the tumor of the mouse.

**Example 3**

Preparation of magnetic ferroferric oxide nanoparticle (HPMA-ES-MION):

20 mL of 2 mg/mL polymaleic acid HPMA ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as HPMA-ES-MION.

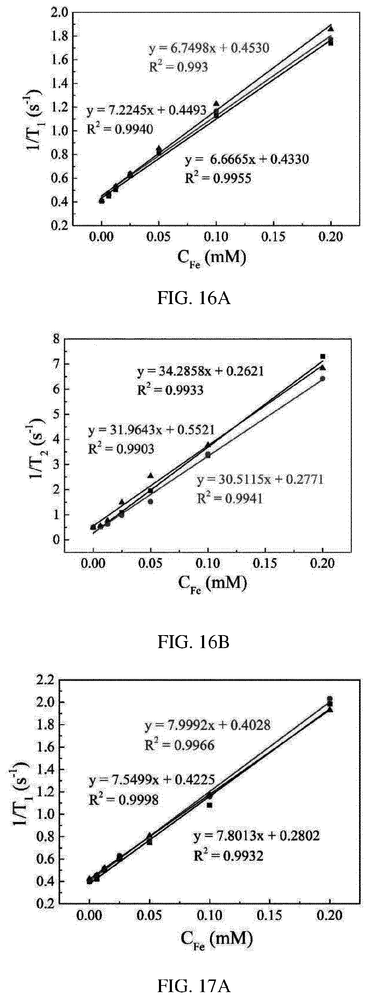

A performance test was carried out on the sample in Example 3. A recovery rate of iron of the sample in Example 3 was calculated to be 87.2%, which indicated that a utilization rate of a raw material was high, so that cost saving was realized. The sample in Example 3 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples HPMA-ES-MION-1, HPMA-ES-MION-2 and HPMA-ES-MION-3 were tested in parallel for three times). As shown in FIG. 16, a change relationship between 1/Ti and a concentration of iron was drawn, and a slope of a fitted line was a magnetic relaxation rate of $T_i$ (i = 1 or 2). Thus, it could be seen that, under the 3.0 T MRI

system, an $r_1$ value was 6.9 ± 0.3 mM$^{-1}$ s$^{-1}$, an $r_2$ value was 32.3 ± 1.9 mM$^{-1}$ s$^{-1}$, and an $r_2/r_1$ ratio was 4.7 ± 0.4 in Example 3. It was proved that the magnetic ferroferric oxide nanoparticle (HPMA-ES-MION) in Example 3 had a high $r_1$ value and a low $r_2/r_1$ ratio, and could be used as a $T_1$-weighted MRI contrast agent with good biocompatibility.

**Example 4**

Preparation of magnetic ferroferric oxide nanoparticle (PEAA-ES-MION):

20 mL of 2 mg/mL poly(2-ethylacrylic acid) PEAA ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M FeSO$_4$ and 0.5 M FeCl$_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PEAA-ES-MION.

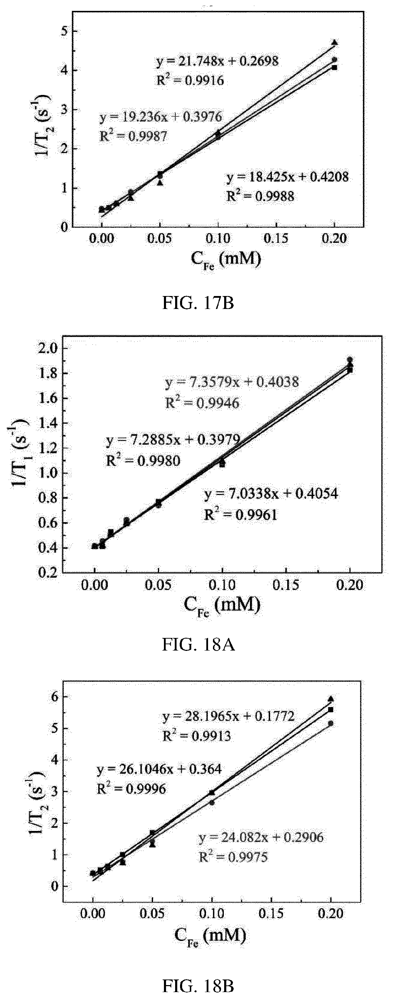

A performance test was carried out on the sample in Example 4. A recovery rate of iron of the sample in Example 4 was calculated to be 89.1%, which indicated that a utilization rate of a raw material was high, so that cost saving was realized. The sample in Example 4 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PEAA-ES-MION-1, PEAA-ES-MION-2 and PEAA-ES-MION-3 were tested in parallel for three times). As shown in FIG. 17, a change relationship between 1/Ti and a concentration of iron was drawn, and a slope of a fitted line was a magnetic relaxation rate of $T_i$ (i = 1 or 2). Thus, it could be seen that, under the 3.0 T MRI system, an $r_1$ value was 7.8 ± 0.2 mM$^{-1}$ s$^{-1}$, an $r_2$ value was 19.8 ± 1.7 mM$^{-1}$ s$^{-1}$, and an $r_2/r_1$ ratio was 2.5 ± 0.3 in Example 4. The results proved that the magnetic ferroferric oxide nanoparticle (PEAA-ES-MION) in Example 4 had an extremely high $r_1$ value and a low $r_2/r_1$ ratio, and could be used as a $T_1$-weighted MRI contrast agent with good biocompatibility.

**Example 5**

Preparation of magnetic ferroferric oxide nanoparticle (PESA-ES-MION):

20 mL of 2 mg/mL polyepoxysuccinic acid PESA ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M FeSO$_4$ and 0.5 M FeCl$_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PES A-ES-MION.

A performance test was carried out on the sample in Example 5. A recovery rate of iron of the sample in Example 5 was calculated to be 93.2%, which indicated that a utilization rate of a raw material was high, so that cost saving was realized. The sample in Example 5 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PESA-ES-MION-1, PESA-ES-MION-2 and PESA-ES-MION-3 were tested in parallel for three times). As shown in FIG. 18, a change relationship between 1/T; and a concentration of iron was drawn, and a slope of a fitted line was a magnetic relaxation rate of $T_i$ (i = 1 or 2). Thus, it could be seen that, under the 3.0 T MRI system, an $r_1$ value was 7.2 ± 0.2 mM$^{-1}$ s$^{-1}$, an $r_2$ value was 26.1 ± 2.1 mM$^{-1}$ s$^{-1}$, and an $r_2/r_1$ ratio was 3.6 ± 0.3 in Example 5. The results proved that the ferroferric oxide nanoparticle (PESA-ES-MION) in Example 5 had an extremely high $r_1$ value and a low $r_2/r_1$ ratio, and could be used as a $T_1$-weighted MRI contrast agent with good biocompatibility.

**Example 6**

Preparation of magnetic ferroferric oxide nanoparticle (ε-PL-ES-MION):

20 mL of 2 mg/mL polylysine ε-PL ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M FeSO$_4$ and 0.5 M FeCl$_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as ε-PL-ES-MION.

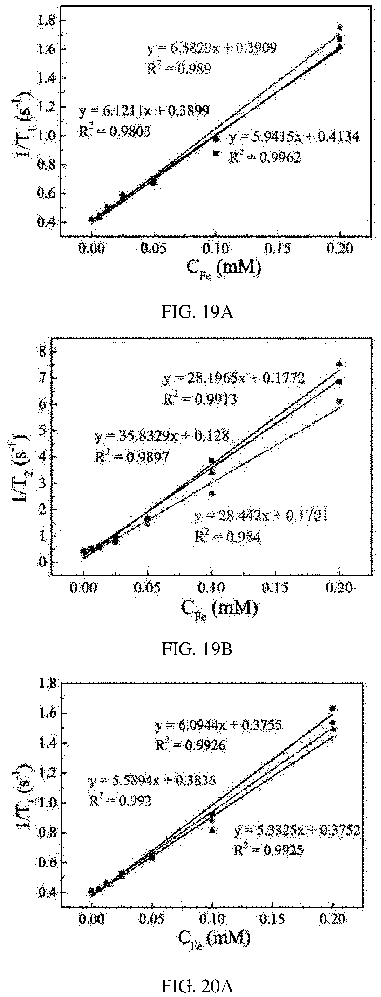

A performance test was carried out on the sample in Example 6. A recovery rate of iron of the sample in Example 6 was calculated to be 82.6%, which indicated that a utilization rate of a raw material was high, so that cost saving was realized. The sample in Example 6 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples ε-PL-ES-MION-1, ε-PL-ES-MION-2 and ε-PL-ES-MION-3 were tested in parallel for three times). As shown in FIG. 19, a change relationship between $1/T_i$ and a concentration of iron was drawn, and a slope of a fitted line was a relaxation rate of $T_i$ ($i$ = 1 or 2). Thus, it could be seen that, under the 3.0 T MRI system, an $r_1$ value was $6.2 \pm 0.3$ mM$^{-1}$ s$^{-1}$, an $r_2$ value was $32.5 \pm 3.8$ mM$^{-1}$ s$^{-1}$, and an $r_2/r_1$ ratio was $5.3 \pm 0.9$ in Example 6. The results proved that the magnetic ferroferric oxide nanoparticle ($\varepsilon$-PL-ES-MION) in Example 6 had an extremely high $r_1$ value and a low $r_2/r_1$ ratio, and could be used as a $T_1$-weighted MRI contrast agent with good biocompatibility.

**Example 7**

Preparation of magnetic ferroferric oxide nanoparticle (PLH-ES-MION):

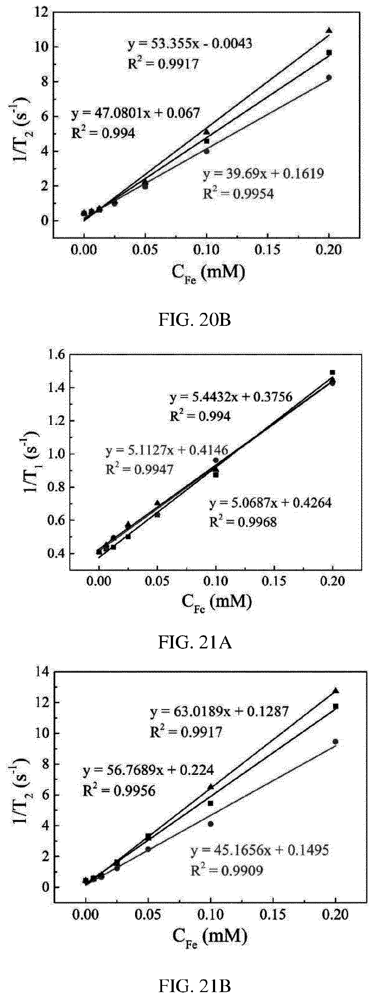

20 mL of 2 mg/mL polyhistidine PLH ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PLH-ES-MION.
A performance test was carried out on the sample in Example 6. A recovery rate of iron of the sample in Example 7 was calculated to be 85.7%, which indicated that a utilization rate of a raw material was high, so that cost saving was realized. The sample in Example 7 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PLH-ES-MION-1, PLH-ES-MION-2 and PLH-ES-MION-3 were tested in parallel for three times). As shown in FIG. 20, a change relationship between $1/T$; and a concentration of iron was drawn, and a slope of a fitted line was a magnetic relaxation rate of $T_i$ ($i$ = 1 or 2). Thus, it could be seen that, under the 3.0 T MRI system, an $r_1$ value was $5.7 \pm 0.4$ mM$^{-1}$ s$^{-1}$, an $r_2$ value was $46.6 \pm 7.0$ mM$^{-1}$ s$^{-1}$, and an $r_2/r_1$ ratio was $8.3 \pm 1.5$ in Example 7. The results proved that the magnetic ferroferric oxide nanoparticle (PLH-ES-MION) in Example 7 had an extremely high $r_1$ value and a low $r_2/r_1$ ratio, and could be used as a $T_1$-weighted MRI contrast agent with good biocompatibility.

**Example 8**

Preparation of magnetic ferroferric oxide nanoparticle (PLR-ES-MION):

20 mL of 2 mg/mL poly-L-arginine PLR ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PLR-ES-MION.
A performance test was carried out on the sample in Example 8. A recovery rate of iron of the sample in Example 8 was calculated to be 83.9%, which indicated that a utilization rate of a raw material was high, so that cost saving was realized. The sample in Example 8 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PLR-ES-MION-1, PLR-ES-MION-2 and PLR-ES-MION-3 were tested in parallel for three times). As shown in FIG. 21, a change relationship between $1/T$; and a concentration of iron was drawn, and a slope of a fitted line was a magnetic relaxation rate of $T_i$ ($i$ = 1 or 2). Thus, it could be seen that, under the 3.0 T MRI system, an $r_1$ value was $5.2 \pm 0.2$ mM$^{-1}$ s$^{-1}$, an $r_2$ value was $55.0 \pm 9.1$ mM$^{-1}$ s$^{-1}$, and an $r_2/r_1$ ratio was $10.6 \pm 1.8$ in Example 8. The results proved that the magnetic ferroferric oxide nanoparticle (PLR-ES-MION) in Example 8 had an extremely high $r_1$ value and a low $r_2/r_1$ ratio, and could be used as a $T_1$-weighted MRI contrast agent with good biocompatibility.

Example 9

Preparation of magnetic ferroferric oxide nanoparticle (PDDA-ES-MION):

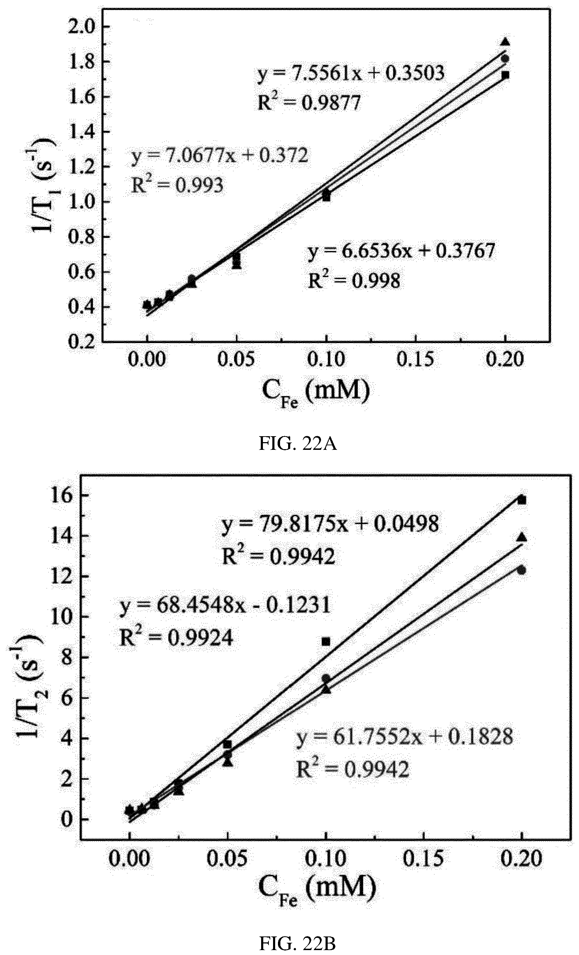

20 mL of 2 mg/mL polydimethyl diallyl ammonium chloride PDDA ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PDDA-ES-MION.
A performance test was carried out on the sample in Example 9. A recovery rate of iron of the sample in Example 9 was calculated to be 90.4%, which indicated that a utilization rate of a raw material was high, so that cost saving was realized. The sample in Example 9 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PDDA-ES-MION-1, PDDA-ES-MION-2 and PDDA-ES-MION-3 were tested in parallel for three times). As shown in FIG. 22, a change relationship between 1/T; and a concentration of iron was drawn, and a slope of a fitted line was a magnetic relaxation rate of $T_i$ (i = 1 or 2). Thus, it could be seen that, under the 3.0 T MRI system, an $r_1$ value was $7.1 \pm 0.5$ mM$^{-1}$ s$^{-1}$, an $r_2$ value was $70.0 \pm 9.1$ mM$^{-1}$ s$^{-1}$, and an $r_2/r_1$ ratio was $9.9 \pm 1.8$ in Example 9. The results proved that the magnetic ferroferric oxide nanoparticle (PDDA-ES-MION) in Example 9 had a high $r_1$ value and a low $r_2/r_1$ ratio, and could be used as a $T_1$-weighted MRI contrast agent with good biocompatibility.

**Example 10**

Preparation of magnetic ferroferric oxide nanoparticle (PSer-ES-MION):

20 mL of 2 mg/mL polyserine PSer ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PSer-ES-MION.

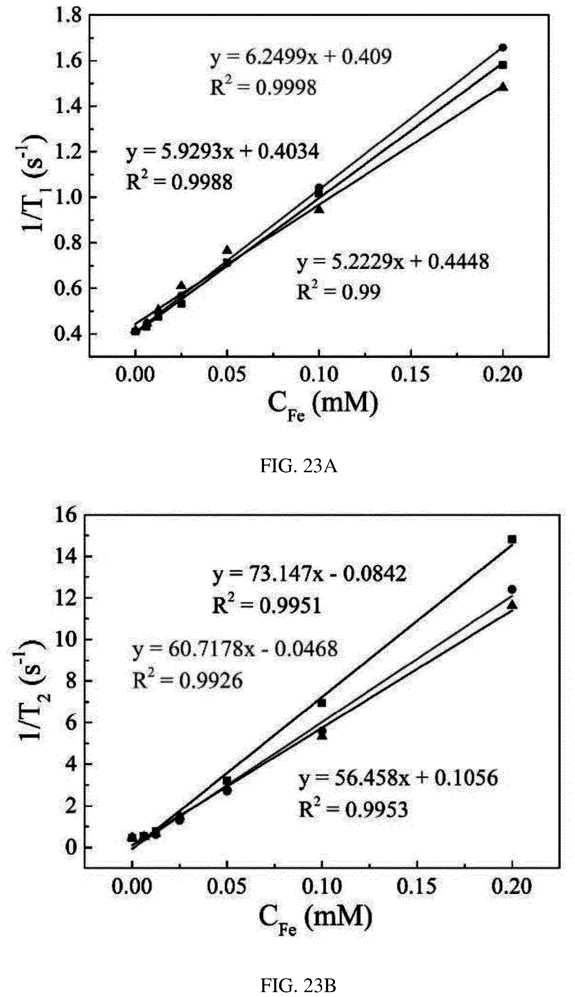

A performance test was carried out on the sample in Example 10. A recovery rate of iron of the sample in Example 10 was calculated to be 88.2%, which indicated that a utilization rate of a raw material was high, so that cost saving was realized. The sample in Example 10 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PSer-ES-MION-1, PSer-ES-MION-2 and PSer-ES-MION-3 were tested in parallel for three times). As shown in FIG. 23, a change relationship between 1/Ti and a concentration of iron was drawn, and a slope of a fitted line was a magnetic relaxation rate of $T_i$ (i = 1 or 2). Thus, it could be seen that, under the 3.0 T MRI system, an $r_1$ value was $5.8 \pm 0.25$ mM$^{-1}$ s$^{-1}$, an $r_2$ value was $63.4 \pm 8.7$ mM$^{-1}$ s$^{-1}$, and an $r_2/r_1$ ratio was $11.0 \pm 1.3$ in Example 10. The results proved that the magnetic ferroferric oxide nanoparticle (PSer-ES-MION) in Example 10 had a high $r_1$ value and a low $r_2/r_1$ ratio, and could be used as a $T_1$-weighted MRI contrast agent with good biocompatibility.

**Example 11**

Preparation of magnetic ferroferric oxide nanoparticle (PThr-ES-MION):

20 mL of 2 mg/mL polythreonine PThr ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PThr-ES-MION.

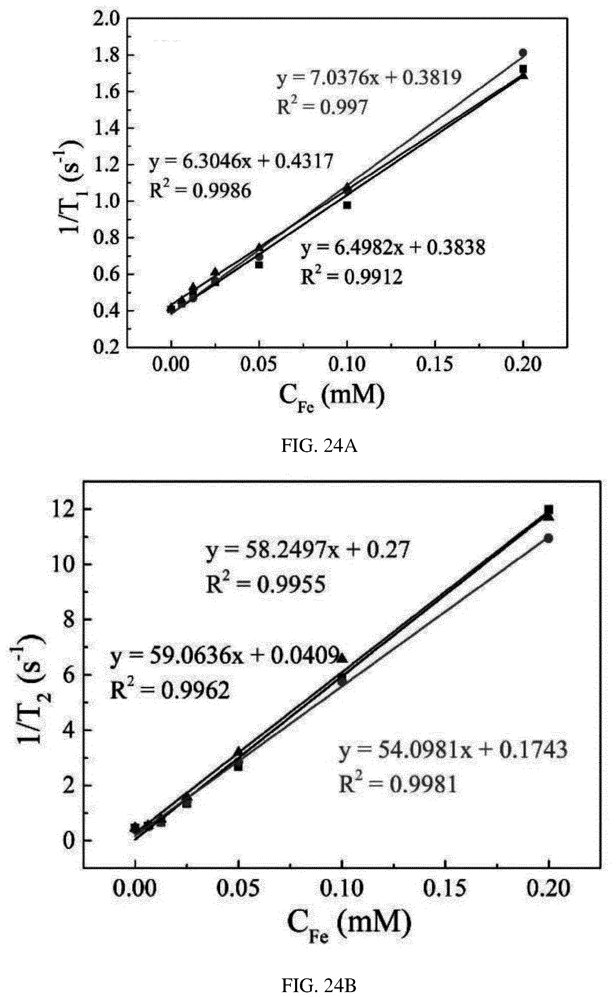

A performance test was carried out on the sample in Example 11. A recovery rate of iron of the sample in Example 11 was calculated to be 85.7%, which indicated that a utilization rate of a raw material was high, so that cost saving was realized. The sample in Example 11 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PThr-ES-MION-1, PThr-ES-MION-2 and PThr-ES-MION-3 were tested in parallel for three times). As shown in FIG. 24, a change relationship between 1/Ti and a concentration of iron was drawn, and a slope of a fitted line was a magnetic relaxation rate of $T_i$ (i = 1 or 2). Thus, it could be seen that, under the 3.0 T MRI system, an $r_1$ value was $6.6 \pm 0.4$ mM$^{-1}$ s$^{-1}$, an $r_2$ value was $57.2 \pm 2.5$ mM$^{-1}$ s$^{-1}$, and an $r_2/r_1$ ratio was $8.7 \pm 0.8$ in Example 11. The results proved that the magnetic ferroferric oxide nanoparticle (PThr-ES-MION) in Example 11 had a high $r_1$ value and a low $r_2/r_1$ ratio, and could be used as a $T_1$-weighted MRI contrast agent with good biocompatibility.

**Example 12**

Preparation of magnetic ferroferric oxide nanoparticle (PTyr-ES-MION):

20 mL of 2 mg/mL polytyrosine PTyr ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PTyr-ES-MION.

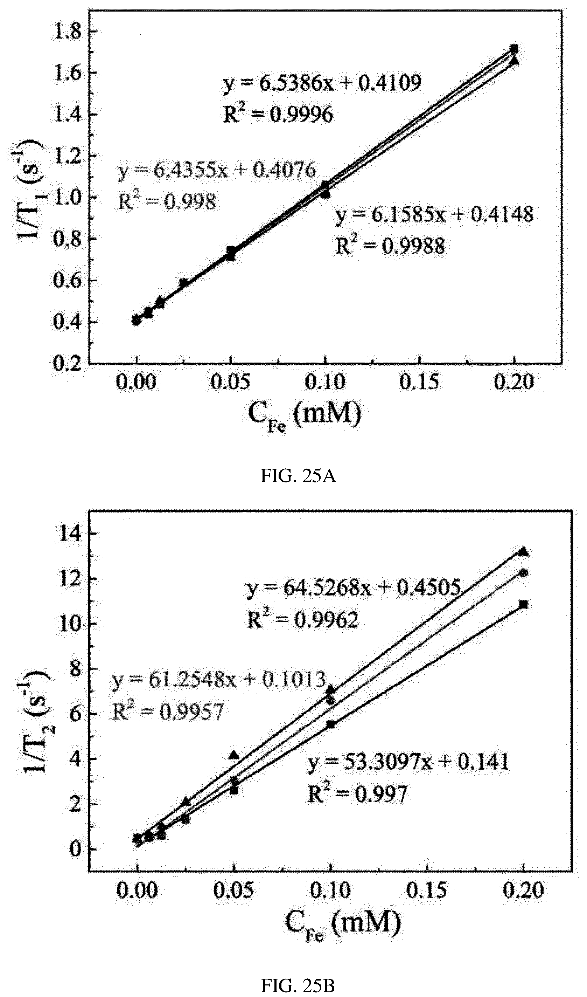

A performance test was carried out on the sample in Example 12. A recovery rate of iron of the sample in Example 12 was calculated to be 83.2%, which indicated that a utilization rate of a raw material was high, so that cost saving was realized. The sample in Example 12 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PTyr-ES-MION-1, PTyr-ES-MION-2 and PTyr-ES-MION-3 were tested in parallel for three times). As shown in FIG. 25, a change relationship between 1/Ti and a concentration of iron was drawn, and a slope of a fitted line was a magnetic relaxation rate of $T_i$ (i = 1 or 2). Thus, it could be seen that, under the 3.0 T MRI system, an $r_1$ value was $6.4 \pm 0.2$ mM$^{-1}$ s$^{-1}$, an $r_2$ value was $59.7 \pm 5.8$ mM$^{-1}$ s$^{-1}$, and an $r_2/r_1$ ratio was $9.4 \pm 1.2$ in Example 12. The results proved that the magnetic ferroferric oxide nanoparticle (PTyr-ES-MION) in Example 12 had a high $r_1$ value and a low $r_2/r_1$ ratio, and could be used as a $T_1$-weighted MRI contrast agent with good biocompatibility.

**Example 13**

Preparation of magnetic ferroferric oxide nanoparticle (TA-ES-MION):

20 mL of 2 mg/mL tannic acid TA ($M_w$ = 1,700) aqueous solution was subjected to bubble deoxygenation with nitrogen in a three-necked flask for 1 hour, and then heated at 100°C to reflux. 0.4 mL of a mixed aqueous solution containing 0.25 M FeSO$_4$ and 0.5 M FeCl$_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as TA-ES-MION.

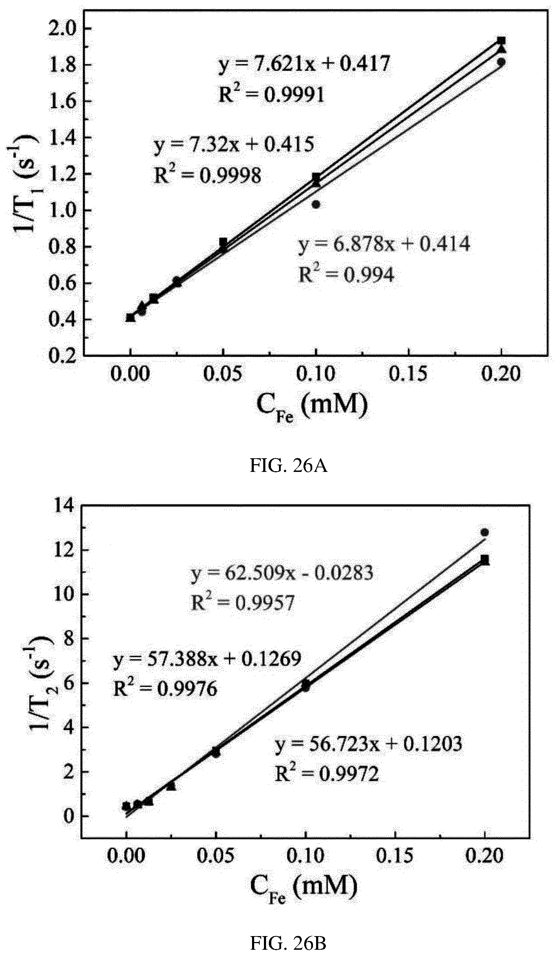

A performance test was carried out on the sample in Example 13. A recovery rate of iron of the sample in Example 13 was calculated to be 92.7%, which indicated that a utilization rate of a raw material was high, so that cost saving was realized. The sample in Example 13 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples TA-ES-MION-1, TA-ES-MION-2 and TA-ES-MION-3 were tested in parallel for three times). As shown in FIG. 26, a change relationship between 1/Ti and a concentration of iron was drawn, and a slope of a fitted line was a magnetic relaxation rate of $T_i$ (i = 1 or 2). Thus, it could be seen that, under the 3.0 T MRI system, an $r_1$ value was $7.3 \pm 0.4$ mM$^{-1}$ s$^{-1}$, an $r_2$ value was $58.9 \pm 3.2$ mM$^{-1}$ s$^{-1}$, and an $r_2/r_1$ ratio was $8.1 \pm 0.8$ in Example 13. The results proved that the magnetic ferroferric oxide nanoparticle (TA-ES-MION) in Example 13 had a high $r_1$ value and a low $r_2/r_1$ ratio, and could be used as a $T_1$-weighted MRI contrast agent with good biocompatibility.

**Example 14**

Preparation of magnetic ferroferric oxide nanoparticle (PolyQ-ES-MION):

20 mL of 2 mg/mL polyglutamine PolyQ ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M FeSO$_4$ and 0.5 M FeCl$_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PolyQ-ES-MION.

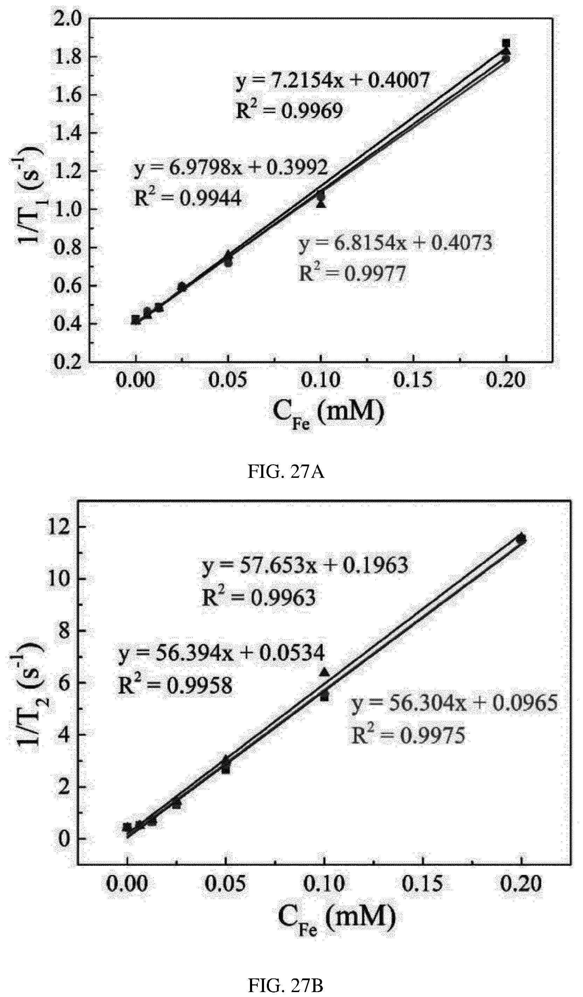

A performance test was carried out on the sample in Example 14. A recovery rate of iron of the sample in Example 14 was calculated to be 87.5%, which indicated that a utilization rate of a raw material was high, so that cost saving was realized. The sample in Example 14 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PolyQ-ES-MION-1, PolyQ-ES-MION-2 and PolyQ-ES-MION-3 were tested in parallel for three times). As shown in FIG. 27, a change relationship between 1/Ti and a concentration of iron was drawn, and a slope of a fitted line was a magnetic relaxation rate of $T_i$ (i = 1 or 2). Thus, it could be seen that, under the 3.0 T MRI system, an $r_1$ value was $7.0 \pm 0.2$ mM$^{-1}$ s$^{-1}$, an $r_2$ value was $56.8 \pm 0.8$ mM$^{-1}$ s$^{-1}$, and an $r_2/r_1$ ratio was $8.1 \pm 0.3$ in Example 14. The results proved that the magnetic ferroferric oxide nanoparticle (PolyQ-ES-MION) in Example 14 had a high $r_1$ value and a low $r_2/r_1$ ratio, and could be used as a $T_1$-weighted MRI contrast agent with good biocompatibility.

**Example 15**

Preparation of magnetic ferroferric oxide nanoparticle (PHEA-ES-MION):

20 mL of 2 mg/mL polyasparamide PHEA ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M FeSO$_4$ and 0.5 M FeCl$_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PHEA-ES-MION.

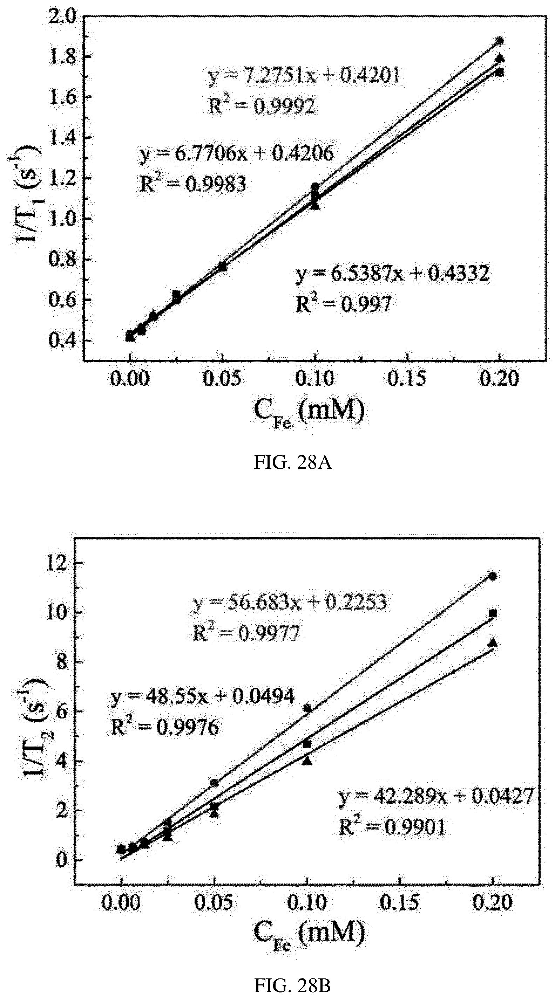

A performance test was carried out on the sample in Example 15. A recovery rate of iron of the sample in Example 15 was calculated to be 85.4%, which indicated that a utilization rate of a raw material was high, so that cost saving was realized. The sample in Example 15 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PHEA-ES-MION-1, PHEA-ES-MION-2 and PHEA-ES-MION-3 were tested in parallel for three times). As shown in FIG. 28, a change relationship between 1/Ti and a concentration of iron was drawn, and a slope of a fitted line was a magnetic relaxation rate of $T_i$ (i = 1 or 2). Thus, it could be seen that, under the 3.0 T MRI system, an $r_1$ value was $6.9 \pm 0.4$ mM$^{-1}$ s$^{-1}$, an $r_2$ value was $49.2 \pm 7.2$ mM$^{-1}$ s$^{-1}$, and an $r_2/r_1$ ratio was $7.2 \pm 0.8$ in Example 15. The results proved that the magnetic ferroferric oxide nanoparticle (PHEA-ES-MION) in Example 15 had a high $r_1$ value and a low $r_2/r_1$ ratio, and could be used as a $T_1$-weighted MRI contrast agent with good biocompatibility.

**Example 16**

Preparation of magnetic ferroferric oxide nanoparticle (PAM-ES-MION):

20 mL of 2 mg/mL polyacrylamide PAM ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PAM-ES-MION.

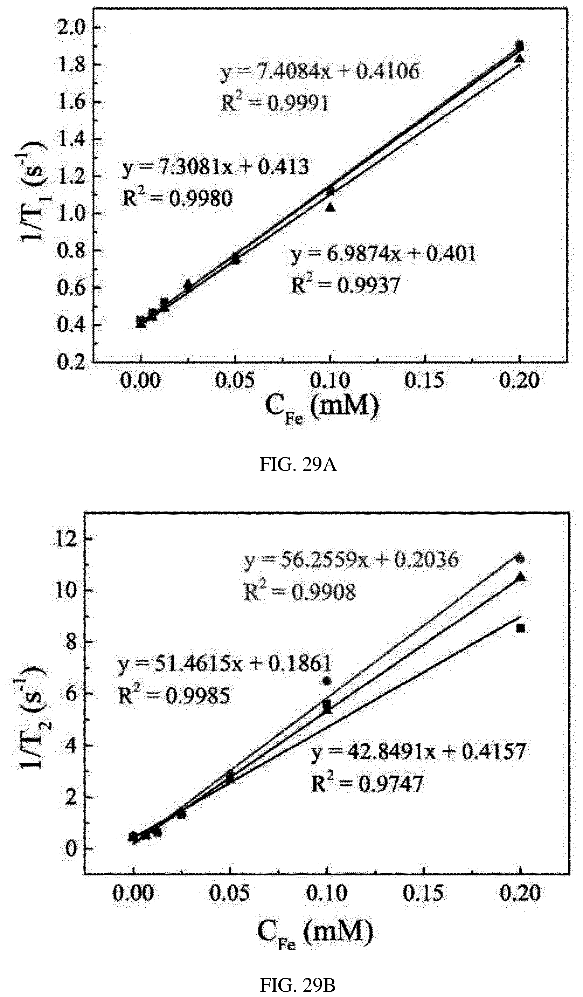

A performance test was carried out on the sample in Example 16. A recovery rate of iron of the sample in Example 16 was calculated to be 84.3%, which indicated that a utilization rate of a raw material was high, so that cost saving was realized. The sample in Example 16 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PAM-ES-MION-1, PAM-ES-MION-2 and PAM-ES-MION-3 were tested in parallel for three times). As shown in FIG. 29, a change relationship between 1/Ti and a concentration of iron was drawn, and a slope of a fitted line was a magnetic relaxation rate of $T_i$ (i = 1 or 2). Thus, it could be seen that, under the 3.0 T MRI system, an $r_1$ value was $7.2 \pm 0.2$ mM$^{-1}$ s$^{-1}$, an $r_2$ value was $50.2 \pm 6.8$ mM$^{-1}$ s$^{-1}$, and an $r_2/r_1$ ratio was $6.9 \pm 0.9$ in Example 16. The results proved that the magnetic ferroferric oxide nanoparticle (PAM-ES-MION) in Example 16 had a high $r_1$ value and a low $r_2/r_1$ ratio, and could be used as a $T_1$-weighted MRI contrast agent with good biocompatibility.

**Example 17**

Preparation of magnetic ferroferric oxide nanoparticle (PMAM-ES-MION):

20 mL of 2 mg/mL polymethacrylamide PMAM ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as PMAM-ES-MION.

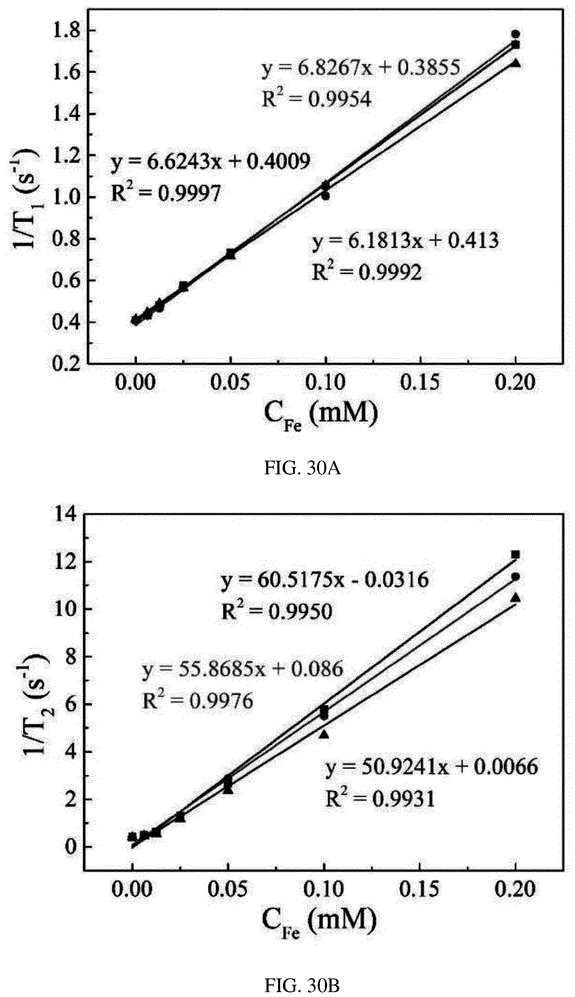

A performance test was carried out on the sample in Example 17. A recovery rate of iron of the sample in Example 17 was calculated to be 90.7%, which indicated that a utilization rate of a raw material was high, so that cost saving was realized. The sample in Example 17 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples PMAM-ES-MION-1, PMAM-ES-MION-2 and PMAM-ES-MION-3 were tested in parallel for three times). As shown in FIG. 30, a change relationship between 1/T; and a concentration of iron was drawn, and a slope of a fitted line was a magnetic relaxation rate of $T_i$ (i = 1 or 2). Thus, it could be seen that, under the 3.0 T MRI system, an $r_1$ value was $6.5 \pm 0.3$ mM$^{-1}$ s$^{-1}$, an $r_2$ value was $55.8 \pm 4.8$ mM$^{-1}$ s$^{-1}$, and an $r_2/r_1$ ratio was $8.5 \pm 0.5$ in Example 17. The results proved that the magnetic ferroferric oxide nanoparticle (PMAM-ES-MION) in Example 17 had a high $r_1$ value and a low $r_2/r_1$ ratio, and could be used as a $T_1$-weighted MRI contrast agent with good biocompatibility.

**Example 18**

Preparation of magnetic ferroferric oxide nanoparticle (HA-ES-MION):

20 mL of 2 mg/mL hyaluronic acid HA ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as HA-ES-MION.

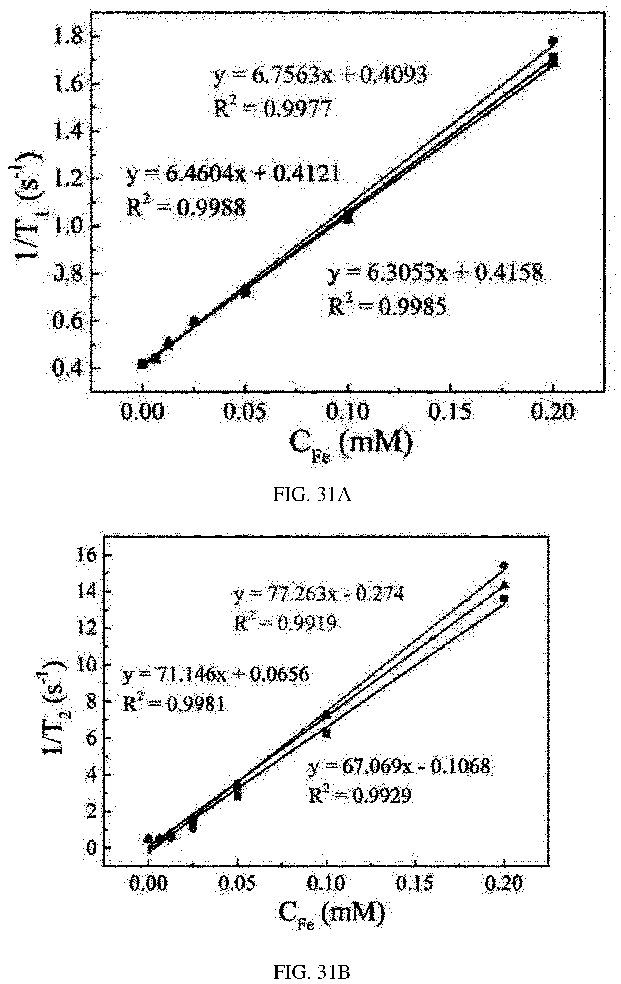

A performance test was carried out on the sample in Example 18. A recovery rate of iron of the sample in Example 18 was calculated to be 89.5%, which indicated that a utilization rate of a raw material was high, so that cost saving was realized. The sample in Example 18 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples HA-ES-MION-1, HA-ES-MION-2 and HA-ES-MION-3 were tested in parallel for three times). As shown in FIG. 31, a change relationship between $1/T_i$ and a concentration of iron was drawn, and a slope of a fitted line was a magnetic relaxation rate of $T_i$ (i = 1 or 2). Thus, it could be seen that, under the 3.0 T MRI system, an $r_1$ value was $6.5 \pm 0.2$ mM-1 s-1, an $r_2$ value was $71.8 \pm 5.1$ mM-1 s-1, and an $r_2/r_1$ ratio was $11.0 \pm 0.6$ in Example 18. The results proved that the magnetic ferroferric oxide nanoparticle (HA-ES-MION) in Example 18 had a high $r_1$ value and a low $r_2/r_1$ ratio, and could be used as a $T_1$-weighted MRI contrast agent with good biocompatibility.

## Example 19

Preparation of magnetic ferroferric oxide nanoparticle (SA-ES-MION):

20 mL of 2 mg/mL sodium alginate SA ($M_w$ = 2,000) aqueous solution was subjected to bubble deoxygenation with nitrogen in a three-necked flask for 1 hour, and then heated at 100°C to reflux. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as SA-ES-MION.

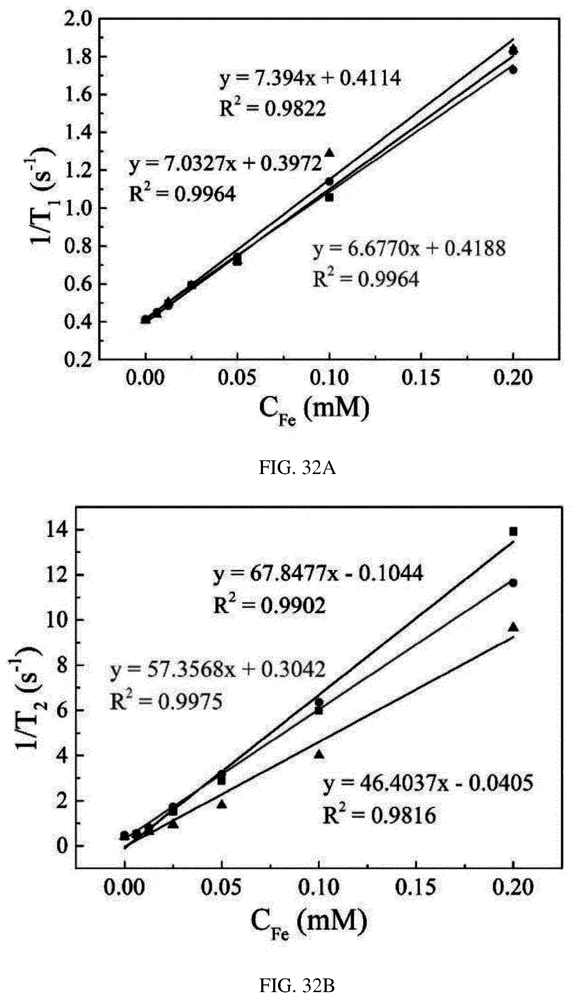

A performance test was carried out on the sample in Example 19. A recovery rate of iron of the sample in Example 19 was calculated to be 89.5%, which indicated that a utilization rate of a raw material was high, so that cost saving was realized. The sample in Example 19 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples SA-ES-MION-1, SA-ES-MION-2 and SA-ES-MION-3 were tested in parallel for three times). As shown in FIG. 32, a change relationship between $1/T_i$ and a concentration of iron was drawn, and a slope of a fitted line was a magnetic relaxation rate of $T_i$ (i = 1 or 2). Thus, it could be seen that, under the 3.0 T MRI system, an $r_1$ value was $7.0 \pm 0.4$ mM-1 s-1, an $r_2$ value was $57.2 \pm 10.7$ mM-1 s-1, and an $r_2/r_1$ ratio was $8.2 \pm 1.7$ in Example 19. The results proved that the magnetic ferroferric oxide nanoparticle (SA-ES-MION) in Example 19 had a high $r_1$ value and a low $r_2/r_1$ ratio, and could be used as a $T_1$-weighted MRI contrast agent with good biocompatibility.

## Example 20

Preparation of magnetic ferroferric oxide nanoparticle ($\gamma$-PGA/PASP-ES-MION):

20 mL of 2 mg/mL polyglutamic acid $\gamma$-PGA/polyaspartic acid PASP (polyglutamic acid has a $M_w$ of 2,000, and polyaspartic acid has a $M_w$ of 7,000 to 8,000) mixture (in a mass ratio of 1: 1) solution was heated to reflux in a three-necked flask, and subjected to bubble deoxygenation with nitrogen for 1 hour. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as $\gamma$-PGA/PASP-ES-MION.

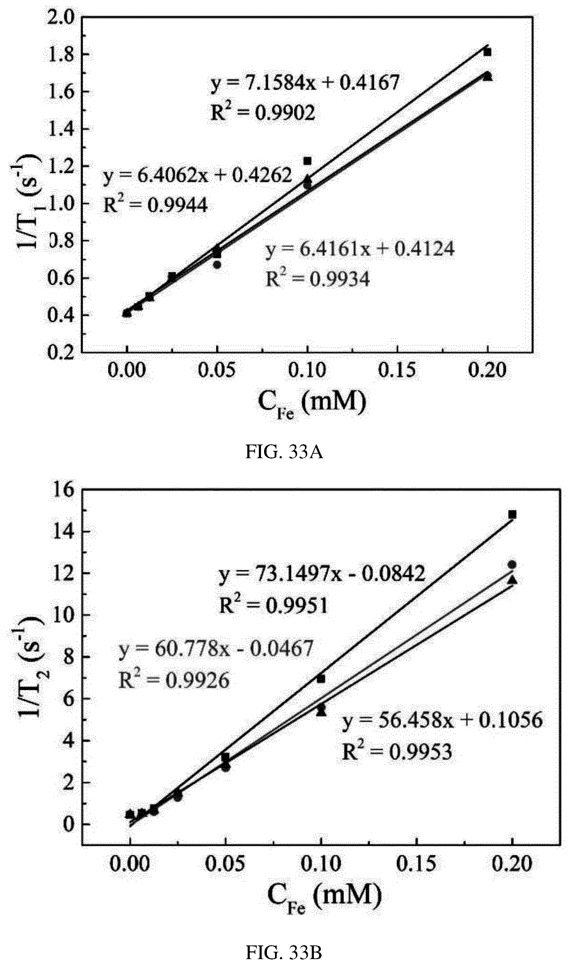

A performance test was carried out on the sample in Example 20. A recovery rate of iron of the sample in Example 20 was calculated to be 93.7%, which indicated that a utilization rate of a raw material was high, so that cost saving was realized. The sample in Example 20 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples $\gamma$-PGA/PASP-ES-MION-1, $\gamma$-PGA/PASP-ES-MION-2 and $\gamma$-PGA/PASP-ES-MION-3 were tested in parallel for three times). As shown in FIG. 33, a change relationship between $1/T_i$ and a concentration of iron was drawn, and a slope of a fitted line was a magnetic relaxation rate of $T_i$ (i = 1 or 2). Thus, it could be seen that, under the 3.0 T MRI system, an $r_1$ value was $6.7 \pm 0.4$ mM-1 s-1, an $r_2$ value was $63.4 \pm 8.7$ mM-1 s-1, and an $r_2/r_1$ ratio was $9.5 \pm 0.7$ in Example 20. The results proved that the magnetic ferroferric oxide nanoparticle ($\gamma$-PGA/PASP-ES-MION) in Example 20 had a high $r_1$ value and a low $r_2/r_1$ ratio, and could be used as a $T_1$-weighted MRI contrast agent with good biocompatibility.

**Example 21**

Preparation of magnetic ferroferric oxide nanoparticle (HPMA/PASP-ES-MION):

20 mL of 2 mg/mL polymaleic acid HPMA/polyaspartic acid PASP (polymaleic acid has a $M_w$ of 2,000, and polyaspartic acid has a $M_w$ of 7,000 to 8,000) mixture (in a mass ratio of 1: 1) solution was heated to reflux in a three-necked flask, and subjected to bubble deoxygenation with nitrogen for 1 hour. 0.4 mL of a mixed aqueous solution containing 0.25 M $FeSO_4$ and 0.5 M $FeCl_3$ was added into the flask, and then added with 6 mL of 1% ammonia water. The mixture reacted for 1 hour under magnetic stirring at 100°C, and was allowed to stand and cooled. The sample after the reaction was dialyzed and purified, and the final sample was marked as HPMA/PASP-ES-MION.

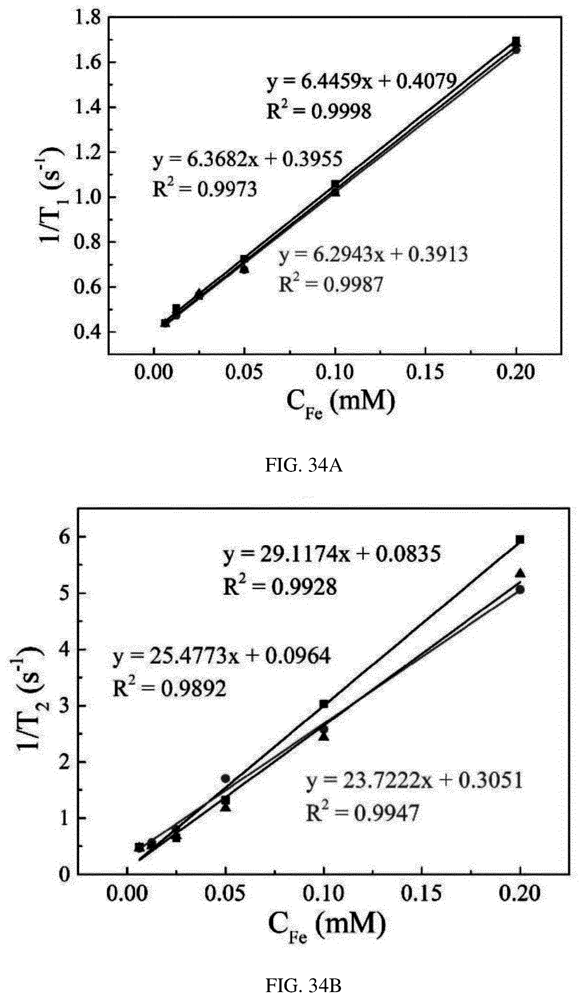

A performance test was carried out on the sample in Example 21. A recovery rate of iron of the sample in Example 21 was calculated to be 90.1%, which indicated that a utilization rate of a raw material was high, so that cost saving was realized. The sample in Example 21 was prepared into 6 aqueous solutions with different concentrations, and in-vitro imaging tests were carried out by a clinical 3.0 T MRI system (Philips, Ingenia) to obtain longitudinal relaxation time $T_1$ and transverse relaxation time $T_2$ (three samples HPMA/PASP-ES-MION-1, HPMA/PASP-ES-MION-2 and HPMA/PASP-ES-MION-3 were tested in parallel for three times). As shown in FIG. 34, a change relationship between $1/T_i$ and a concentration of iron was drawn, and a slope of a fitted line was a magnetic relaxation rate of $T_i$ (i = 1 or 2). Thus, it could be seen that, under the 3.0 T MRI system, an $r_1$ value was 6.4 $\pm$ 0.1 mM-1 s$^{-1}$, an $r_2$ value was 26.1 $\pm$ 2.8 mM-1 s-1, and an $r_2/r_1$ ratio was 4.1 $\pm$ 0.4 in Example 21. The results proved that the magnetic ferroferric oxide nanoparticle (HPMA/PASP-ES-MION) in Example 21 had a high $r_1$ value and a low $r_2/r_1$ ratio, and could be used as a $T_1$-weighted MRI contrast agent with good biocompatibility.

The above examples are the preferred implementations of the present disclosure, but the implementations of the present disclosure are not limited by the above examples. Any other changes, modifications, substitutions, combinations, and simplifications made without departing from the spirit and principle of the present disclosure should be equivalent substitute modes, and should be included in the scope of protection of the present disclosure.

**Claims**

1. A magnetic ferroferric oxide nanoparticle, comprising ferroferric oxide and a hydrophilic macromolecule, wherein the ferroferric oxide and the hydrophilic macromolecule are in at least one of the following relationships (1) and (2): (1) the hydrophilic macromolecule is adsorbed on a surface of the ferroferric oxide; and (2) the ferroferric oxide and the hydrophilic macromolecule are mutually embedded or occluded; and
the magnetic ferroferric oxide nanoparticle simultaneously has the following properties 1) to 4):

   1) an average particle size is greater than 2 nm and less than 5 nm;
   2) an electrokinetic potential is less than or equal to -10 mV;
   3) a hydrodynamic diameter is less than or equal to 20 nm; and
   4) a longitudinal magnetic relaxation rate $r_1$ value under a magnetic field intensity of 3.0 T is greater than 5 mM$^{-1}$ s$^{-1}$; and a longitudinal magnetic relaxation rate $r_1$ value under a magnetic field intensity of 1.0 T is greater than 10 mM$^{-1}$ s$^{-1}$.

2. The magnetic ferroferric oxide nanoparticle according to claim 1, wherein the hydrophilic macromolecule comprises any one or a copolymer or mixture of several of a carboxylic acid-containing macromolecule, an amino-containing macromolecule, a hydroxyl-containing macromolecule, an amide-containing macromolecule and a polysaccharide.

3. The magnetic ferroferric oxide nanoparticle according to claim 2, wherein the carboxylic acid-containing macromolecule comprises any one or more of polyglutamic acid, polyaspartic acid, polymaleic acid, poly(2-ethylacrylic acid) and polyepoxysuccinic acid.

4. The magnetic ferroferric oxide nanoparticle according to claim 2, wherein the amino-containing macromolecule comprises any one or more of polylysine, polyhistidine, poly-L-arginine and polydimethyl diallyl ammonium chloride.

5. The magnetic ferroferric oxide nanoparticle according to claim 2, wherein the hydroxyl-containing macromolecule comprises any one or more of polyserine, polythreonine, polytyrosine and tannic acid.

6. The magnetic ferroferric oxide nanoparticle according to claim 2, wherein the amide-containing macromolecule comprises any one or more of polyglutamine, polyasparamide, polyacrylamide and polymethacrylamide.

7. The magnetic ferroferric oxide nanoparticle according to claim 2, wherein the polysaccharide comprises one or two of hyaluronic acid and sodium alginate.

8. A preparation method for the magnetic ferroferric oxide nanoparticle according to any one of claims 1 to 7, comprising a step of: coprecipitating ferrous ions and ferric ions to form the magnetic ferroferric oxide nanoparticle by using the hydrophilic macromolecule as a stabilizer.

9. The preparation method according to claim 8, wherein the preparation method for the magnetic ferroferric oxide nanoparticle comprises the following steps of: heating a hydrophilic macromolecule solution, then mixing the hydrophilic macromolecule solution after being subjected to heating with an iron ion mixed solution comprising ferrous ions and ferric ions for a coordination reaction, and then adding an alkali liquor for a coprecipitation reaction to obtain the magnetic ferroferric oxide nanoparticle.

10. Use of the magnetic ferroferric oxide nanoparticle according to any one of claims 1 to 7 in preparing a magnetic resonance imaging contrast agent.

FIG. 1A

$\overline{d} = 3.79$ nm

FIG. 1B

FIG. 2A

FIG. 2B

$$y = 5.6545x + 0.4119$$
$$R^2 = 0.9992$$

$$y = 5.7201x + 0.4025$$
$$R^2 = 0.9995$$

$$y = 5.7118x + 0.4035$$
$$R^2 = 0.9989$$

FIG. 3A

$$y = 71.8045x - 0.0237$$
$$R^2 = 0.9994$$

$$y = 69.5980x + 0.0154$$
$$R^2 = 0.9995$$

$$y = 69.0721x - 0.0105$$
$$R^2 = 0.9996$$

FIG. 3B

FIG. 4

FIG. 5A

FIG. 5B

FIG. 6

FIG. 7A

FIG. 7B

FIG. 8

FIG. 9A

FIG. 9B

FIG. 10A

FIG. 10B

FIG. 11A

FIG. 11B

FIG. 12A

FIG. 12B

FIG. 13

FIG. 14A

FIG. 14B

FIG. 15

FIG. 16A

FIG. 16B

FIG. 17A

FIG. 17B

FIG. 18A

FIG. 18B

FIG. 19A

FIG. 19B

FIG. 20A

FIG. 20B

FIG. 21A

FIG. 21B

$y = 7.5561x + 0.3503$
$R^2 = 0.9877$

$y = 7.0677x + 0.372$
$R^2 = 0.993$

$y = 6.6536x + 0.3767$
$R^2 = 0.998$

FIG. 22A

$y = 79.8175x + 0.0498$
$R^2 = 0.9942$

$y = 68.4548x - 0.1231$
$R^2 = 0.9924$

$y = 61.7552x + 0.1828$
$R^2 = 0.9942$

FIG. 22B

FIG. 23A

FIG. 23B

FIG. 24A

FIG. 24B

y = 6.5386x + 0.4109
R² = 0.9996

y = 6.4355x + 0.4076
R² = 0.998

y = 6.1585x + 0.4148
R² = 0.9988

FIG. 25A

y = 64.5268x + 0.4505
R² = 0.9962

y = 61.2548x + 0.1013
R² = 0.9957

y = 53.3097x + 0.141
R² = 0.997

FIG. 25B

FIG. 26A

FIG. 26B

FIG. 27A

FIG. 27B

y = 7.2751x + 0.4201
R² = 0.9992

y = 6.7706x + 0.4206
R² = 0.9983

y = 6.5387x + 0.4332
R² = 0.997

FIG. 28A

y = 56.683x + 0.2253
R² = 0.9977

y = 48.55x + 0.0494
R² = 0.9976

y = 42.289x + 0.0427
R² = 0.9901

FIG. 28B

$$y = 7.4084x + 0.4106$$
$$R^2 = 0.9991$$

$$y = 7.3081x + 0.413$$
$$R^2 = 0.9980$$

$$y = 6.9874x + 0.401$$
$$R^2 = 0.9937$$

FIG. 29A

$$y = 56.2559x + 0.2036$$
$$R^2 = 0.9908$$

$$y = 51.4615x + 0.1861$$
$$R^2 = 0.9985$$

$$y = 42.8491x + 0.4157$$
$$R^2 = 0.9747$$

FIG. 29B

FIG. 30A

FIG. 30B

FIG. 31A

FIG. 31B

FIG. 32A

FIG. 32B

FIG. 33A

FIG. 33B

FIG. 34A

FIG. 34B

## INTERNATIONAL SEARCH REPORT

| International application No. |
| --- |
| **PCT/CN2022/119469** |

### A. CLASSIFICATION OF SUBJECT MATTER

A61K 49/18(2006.01)i; A61K 49/14(2006.01)i; A61K 49/12(2006.01)i; B82Y 5/00(2011.01)i; B82Y 40/00(2011.01)i

According to International Patent Classification (IPC) or to both national classification and IPC

### B. FIELDS SEARCHED

Minimum documentation searched (classification system followed by classification symbols)

A61K; B82Y

Documentation searched other than minimum documentation to the extent that such documents are included in the fields searched

Electronic data base consulted during the international search (name of data base and, where practicable, search terms used)

CNABS; WPABSC; DWPI; WPABS; VEN; CNTXT; EPTXT; USTXT; WOTXT; CNKI; 中国药物专利数据库, CTCMPD; 百度学术搜索, BAIDU SCHOLAR SEARCH; ISI-WEB OF SCIENCE; Pubmed: 四氧化三铁, 氧化铁, 磁性, 纳米粒, 亲水, 聚谷氨酸, 聚天冬氨酸, 聚马来酸, 聚(2-乙基丙烯酸), 聚环氧琥珀酸, 聚赖氨酸, 聚组氨酸, 聚精氨酸, 聚二甲基二烯丙基氯化铵, 聚丝氨酸, 聚苏氨酸, 聚酪氨酸, 单宁酸, 聚谷氨酰胺, 聚天冬酰胺, 聚丙烯酰胺, 聚甲基丙烯酰胺, 透明质酸, 海藻酸, T1对比剂, Ferric oxide, ion oxide, Fe3O4, USPIO, Magnetic, nanoparticle, hydrophilic, polyglutamic acid, polyaspartic acid, polymaleic acid, poly (2-ethylacrylic acid), polyepoxysuccinic acid, polylysine, polyhistidine, polyarginine, polydimethyldiallylammonium chloride, polyserine, polythreonine, polytyrosine, tannic acid, polyglutamine, polypolyacrylamide, polyaspartic acid, polypolyacrylamide, Polymethylacrylamide, hyaluronic acid, alginate, T1 contrast agent

### C. DOCUMENTS CONSIDERED TO BE RELEVANT

| Category* | Citation of document, with indication, where appropriate, of the relevant passages | Relevant to claim No. |
| --- | --- | --- |
| PX | CN 114306650 A (SOUTHERN MEDICAL UNIVERSITY) 12 April 2022 (2022-04-12) claims 1-10 | 1-10 |
| X | CN 104758956 A (NATIONAL CENTER FOR NANOSCIENCE AND TECHNOLOGY OF CHINA) 08 July 2015 (2015-07-08) description, paragraphs 7-48 and 61, and figure 3 | 1-2, 7-10 |
| Y | CN 104758956 A (NATIONAL CENTER FOR NANOSCIENCE AND TECHNOLOGY OF CHINA) 08 July 2015 (2015-07-08) description, paragraphs 7-48 and 61, and figure 3 | 3-6 |
| Y | CN 111330023 A (CIXI INSTITUTE OF BIOMEDICAL ENGINEERING, NINGBO INSTITUTE OF INDUSTRIAL TECHNOLOGY, CHINESE ACADEMY OF SCIENCES; NINGBO INSTITUTE OF MATERIALS TECHNOLOGY AND ENGINEERING, CHINESE ACADEMY OF SCIENCES) 26 June 2020 (2020-06-26) description, paragraphs 8-118 | 3-6 |

☑ Further documents are listed in the continuation of Box C.    ☑ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **05 December 2022** | **21 December 2022** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**EP 4 467 161 A1**

| | INTERNATIONAL SEARCH REPORT | International application No. |
|---|---|---|
| | | **PCT/CN2022/119469** |

**C.     DOCUMENTS CONSIDERED TO BE RELEVANT**

| Category* | Citation of document, with indication, where appropriate, of the relevant passages | Relevant to claim No. |
|---|---|---|
| A | MA, Hui-Li et al.. "Preparation and characterization of superparamagnetic iron oxide nanoparticles stabilized by alginate." <br> *The International Journal of Pharmaceutics,* Vol. 333, No. 1-2, 21 March 2007 (2007-03-21), ISSN: 0378-5173, <br> pages 177-186 | 1-10 |
| A | XU, Xiaodan et al.. "Glutathione-Responsive Magnetic Nanoparticles for Highly Sensitive Diagnosis of Liver Metastases." <br> *Nano Lett.,* Vol. 21, No. 5, 10 March 2021 (2021-03-10), ISSN: 1530-6992, <br> pages 2199-2206 | 1-10 |

Form PCT/ISA/210 (second sheet) (January 2015)

**INTERNATIONAL SEARCH REPORT**
Information on patent family members

International application No.

**PCT/CN2022/119469**

| Patent document cited in search report | | | Publication date (day/month/year) | Patent family member(s) | | | Publication date (day/month/year) |
|---|---|---|---|---|---|---|---|
| CN | 114306650 | A | 12 April 2022 | None | | | |
| CN | 104758956 | A | 08 July 2015 | None | | | |
| CN | 111330023 | A | 26 June 2020 | WO | 2021190573 | A1 | 30 September 2021 |

Form PCT/ISA/210 (patent family annex) (January 2015)